# EUROPEAN PATENT SPECIFICATION

(11) **EP 1 381 864 B1**
(45) Date of publication and mention of the grant of the patent: **27.08.2008**
(21) Application number: 02717869.8
(22) Date of filing: 23.04.2002
(51) Int. Cl.: G01N 33/569

(54) **DETECTION OF CANDIDA**
CANDIDA-NACHWEIS
DETECTION DU CANDIDA

(30) Priority: 25.04.2001 US 841188
(43) Date of publication of application: 21.01.2004
(73) Proprietor: Rockeby Biomed (Australia) Pty Ltd., Bentley, Western Australia 6102 (AU)
(72) Inventor: WARMINGTON, John, Willetton, Western Australia 6155 (AU); BALLANTYNE, Denis, Kewdale, Western Australia 6105 (AU)
(74) Representative: Williams, Aylsa
(86) International application number: PCT/AU2002/000507
(87) International publication number: WO 2002/088741

(56) References cited:
- EP-A1- 1 026 245
- EP-B1- 0 145 333
- US-A- 4 670 382
- US-A- 4 806 465
- US-A- 5 545 525
- SINCLAIR A L ET AL: "A RAPID ELISA FOR IGG ANTIBODIES TO CANDIDA-ALBICANS PRELIMINARY STUDIES IN DUODENAL ULCER PATIENTS" MEDICAL LABORATORY SCIENCES, vol. 44, no. 2, 1987, pages 137-140, XP009029592 ISSN: 0308-3616
- BALLANTYNE DENIS S ET AL: "Purification of native enolase from medically important Candida species" BIOTECHNOLOGY AND APPLIED BIOCHEMISTRY, vol. 31, no. 3, June 2000 (2000-06), pages 213-218, XP002277458 ISSN: 0885-4513
- SYVERSON R E ET AL: "INCREASING THE PREDICTIVE VALUE POSITIVE OF THE PRECIPITIN TEST FOR THE DIAGNOSIS OF DEEP SEATED CANDIDIASIS" AMERICAN JOURNAL OF CLINICAL PATHOLOGY, vol. 70, no. 5, 1978, pages 826-831, XP009029662 ISSN: 0002-9173
- STROCKBINE N A ET AL: "IDENTIFICATION AND MOLECULAR WEIGHT CHARACTERIZATION OF ANTIGENS FROM CANDIDA-ALBICANS THAT ARE RECOGNIZED BY HUMAN SERA" INFECTION AND IMMUNITY, vol. 43, no. 2, 1984, pages 715-721, XP002277459 ISSN: 0019-9567
- DE REPENTIGNY L: "SERODIAGNOSIS OF CANDIDIASIS ASPERGILLOSIS AND CRYPTOCOCCOSIS" CLINICAL INFECTIOUS DISEASES, vol. 14, no. SUPPL. 1, 1992, pages S11-S22, XP009029578 SYMPOSIUM ON FOCUS ON FUNGAL INFECTIONS: AN UPDATE ON DIAGNOSIS AND TREATMENT, PHOENIX, ARIZONA, USA ISSN: 1058-4838
- RICHARDSON M.D. ET AL.: 'Further evaluation of factors affecting a rapid ELISA procedure for detection of IgG antibodies to candida albicans' MYCOPATHOLOGIA vol. 82, 1983, pages 167 - 173, XP009029595
- KARWOWSKA W. ET AL.: 'Antibody levels to major cytoplasmic antigens isolated from standard and patient strains' ANN. IMMUNOL. (INST. PASTEUR) vol. 135D, 1984, pages 145 - 150, XP009029596
- BENBOUZID A. AND GUINET R.: 'Purification of soluble specific antigens of systemic candidiasis by antibody affinity chromatography' JOURNAL OF CHROMATOGRAPHY vol. 306, 1984, pages 117 - 124, XP009029597
- MORHART M. ET AL.: 'Evaluation of enzyme immunoassay for candida cytoplasmic antigens in neutropenic cancer patients' JOURNAL OF CLINICAL MICROBIOLOGY vol. 32, no. 3, 1994, pages 766 - 776, XP002977375
- ARAJ G.F. ET AL.: 'Diagnostic value of the enzyme-linked immunosorbent assay for detection of candida albicans cytoplasmic antigen in sera of cancer patients' JOURNAL OF CLINICAL MICROBIOLOGY vol. 16, no. 1, 1982, pages 46 - 52, XP002978809
- ASHLEY C. ET AL.: 'Release of candida albicans yeast antigens upon interaction with human neutrophils in vitro' J. MED. MICROBIOL. vol. 46, 1997, pages 747 - 755, XP009029598
- HOPWOOD V. ET AL.: 'A comparison of methods for the detection of candida antigens. Evaluation of a new latex reagent' JOURNAL OF IMMUNOLOGICAL METHODS vol. 80, 1985, pages 199 - 210, XP002977370
- MAUCH H. AND BROMBACH J.: 'Analysis of a solid-phase radioimmunoassay for antibodies to cytoplasmic antigen fractions of candida albicans' JOURNAL OF IMMUNOLOGICAL METHODS vol. 43, 1981, pages 181 - 192, XP002977369
- ODDS F.C. AND EVANS E.G.V.: 'Distribution of pathogenic yeasts and humoral antibodies to candida among hospital in patients' J. CLIN. PHATHOL. vol. 33, 1980, pages 750 - 756, XP009029621
- HOLLIDAY M.G.: 'Candida albicans cytoplasmic antigens for use in serodiagnosis of systemic candidiasis' MEDICAL LABORATORY SCIENCES vol. 38, 1981, pages 285 - 288, XP009029622
- GREENFIELD R.A. AND JONES M.J.: 'Purification and characterization of a major cytoplasmic antigen of candida albicans' INFECTION AND IMMUNITY vol. 34, no. 2, 1981, pages 469 - 477, XP002977361

## Description

### FIELD OF THE INVENTION

The present invention relates to a method and a means of diagnosing *Candida* infection. In particular the present invention relates to a method of diagnosing *Candida* infection which is both sensitive and rapid.

### BACKGROUND OF THE INVENTION

*Candida* is the most commonly identified causative agent of oral or vaginal thrush. However, over the last few decades *Candida* has emerged as a significant cause of life-threatening infections in hospital patients. Ironically the increasing incidence of these "invasive" or "systemic" *Candida* infections has been advances in modern medicine. Patients that are now surviving major injuries, surgery, cancers and organ transplants are vulnerable to life-threatening *Candida* infections. In the United States, *Candida* is now the fourth most common cause of blood infections in hospitals.

The major problem with systemic *Candida* infections is that there are few definitive clinical signs or symptoms. Treatment is largely based on suspicion rather than a definitive diagnosis. Even with the availability of anti-fungal drugs such as fluconazole a high mortality rate (30 to 70%) is associated with systemic *Candida* infections. The high rate of mortality is largely due to the rapid onset of infection and a rapidly fatal outcome. Without an accurate diagnosis the infection often goes unnoticed until it is too late to effectively treat. This has led to a comment by clinicians that *Candida* infections are usually diagnosed at autopsy. Accordingly, there is a need for a rapid diagnostic assay that is capable of early diagnosis of *Candida* infection so that appropriate treatment may be instituted thereby reducing the mortality rate.

The main difficulty in the diagnosis of *Candida* infections is that being a commensal, mere isolation of *Candida* from body surfaces, or orifices, is not diagnostic of an infection. Culture of *Candida* from blood or deep tissue is still the main method of diagnosis of systemic *Candida* infections. However, it can take several days for a culture to become positive, by then it may be too late to effectively treat the infection. Also, false positives may occur due to contamination from superficial body sites. Of more importance, is the observation that in up to fifty percent of autopsy proven cases of systemic candidiasis, blood cultures were negative and therefore of no diagnostic value.

Nuclear magnetic resonance (NMR) and radioisotope scanning have been used to detect *Candida* infections in tissues and organs. However, those methods are not useful for early diagnosis.

Recently analysis of the *Candida* metabolite arabinitol was proposed as a diagnostic tool. However, as arabinitol is produced by the human body, further clinical studies have cast doubt on its value.

The polymerase chain reaction (PCR) has also been used in the diagnosis of invasive *Candida* infections. However, PCR has not established itself as a useful diagnostic method for *Candida* for the same reasons as outlined above ie *Candida* is a ubiquitously present microorganism and false positives, due to superficial contamination, are prevalent.

Immunoassays are the established procedures for the diagnosis of many types of infectious diseases. Immunoassays have the advantage that they are rapid and have a standardised assay format. Immunoassays can be designed to either detect *Candida* antigens, or host antibodies reactive against *Candida* antigens. Several immunoassays are commercially available for the detection of *Candida* antigens in sera or other body fluids. However, these assays lack either sensitivity or specificity or both.

Sinclair et al Medical Laboratory Sciences (1987) 44, 137-140 discloses a rapid enzyme-linked immunosorbent assay (ELISA) for IgG antibodies to *Candida albicans,* in particular preliminary studies in duodenal ulcer patients.

Mauch & Brombach, J. of Immunological Methods, 43 (1981) 181-192 discloses the analysis of a solid-phase radioimmunoassay for antibodies to cytoplasmic antigen fractions of *Candida albicans.*

Immunoassays have been developed based on the detection of immunodominant *Candida* antigens. *Candida* mannan is a highly immunogenic cell wall antigen. However, as *Candida* is a commensal, most individuals have antibody to *Candida* mannan, so its usefulness in the diagnosis of systemic infection is limited. The applicant has now surprisingly found that a more discriminatory assay for *Candida* than previously used is the detection of cytoplasmic antigen. The advantage of this diagnostic assay is that antibody to this cytoplasmic antigen is only produced in response to an actual infection. The applicant has further demonstrated that the use of a combination of cytoplasmic antigen with other antigens is very predicative of *Candida* infection.

Accordingly, the present invention overcomes or at least alleviates the problems normally associated with diagnosing *Candida* infection.

### SUMMARY OF THE INVENTION

In its most general aspect, the invention disclosed herein provides a simple and rapid method for diagnosis of *Candida* infection. The method of diagnosis of *Candida* infection may be used to screen large numbers of samples for possible infection.

Accordingly, in one aspect, the invention provides a method of diagnosing *Candida albicans* infection, comprising the steps of:
a) preparing an antigen composition by a method comprising filtration and organic extraction of yeast cells of *Candida albicans* to produce a soluble cytoplasmic protein fraction, the antigen composition being mannose depleted and comprising antigens of molecular weights 55 kDa, 30 kDa, 20 kDa and four of 35 to 45 kDa (when determined by SDS-PAGE) for detecting antibodies to *Candida albicans;*
b) contacting said antigen composition with a biological sample obtained from a subject at risk of, or suspected to be suffering from, *Candida albicans* infection; and
c) using a detection system to determine if antibodies from the biological sample are bound to said antigen composition by means of an antigen/antibody reaction.

Antibody levels may be measured using known techniques of immunology including enzyme-linked immunoassay (ELISA or EIA), biligand binding (sandwich technique), fluorometric assay, chemiluminescent assay, immunochromatography, radialimmunodiffusion or radioimmunoassay (RIA). ELISA, immunochromatography or chemiluminescent assay methods are particularly preferred, since these are quick, sensitive, and specific, and are readily automated for large-scale use. These methods also provide quantitative determinations.

The diagnostic method utilises antigens expressed by *Candida,* especially cytoplasmic antigen. The antigens isolated from *Candida* as disclosed herein may, in certain embodiments of the diagnostic method of the present invention, be immobilised on an inert surface, embedded in a gel, or may be conjugated to a molecule which imparts colour, fluorescence or radioactivity to the antigen.

A method for assessing the prognosis of *Candida* infection, comprising the steps of measuring the levels of antibody to *Candida* cytoplasmic antigen in a biological sample is taught herein.

Persons skilled in the art will appreciate that the techniques disclosed herein may be used on any type of biological sample. Preferable the biological sample is selected from the group consisting of bone marrow, plasma, spinal fluid, lymph fluid, the external sections of the skin from respiratory, intestinal, and genitourinary tracts, tears, saliva, milk, blood; both whole blood and sera, blood cells, tumours and organs. Most preferably the biological sample is sera.

Biological samples that may be analysed by the method of the present invention can also be obtained via swabs, shunts or the like. The biological samples may be analysed directly, or may be treated prior to testing by, for example, concentration or pH adjustment.

A method of detecting the presence or absence of a *Candida* antibody comprising the steps of:
a). exposing a biological sample, which may include a *Candida* antibody, to an isolated cytoplasmic *Candida* antigen; and
b). detecting the reaction between antibody and antigen is taught herein.

In an especially preferred embodiment of the present invention the diagnostic assay further utilises other *Candida* antigens in combination with the cytoplasmic antigen. In particular the cell wall antigen (including mannose) and/or purified immunodominant antigen (enolase) are utilised.

A method of diagnosing *Candida* infection, comprising the steps of:
a). obtaining a biological sample from a subject at risk of, or suspected to be suffering from, *Candida* infection, and
b). measuring the levels of antibody present in the biological sample to *Candida* cytoplasmic antigen in combination with measuring the levels of antibody to either cell wall antigen or immunodominant antigen (enolase) or both is taught herein.

The reagents and means of diagnosis of the present invention may also be embodied in a kit for use in a diagnostics laboratory or may be adapted and automated for analysing large numbers of samples at a central receiving centre.

A method of preparing a cytoplasmic antigen comprising the step of removing lipoproteins by chloroform extraction is taught herein.

### BRIEF DESCRIPTION OF THE FIGURES

Figure 1 shows a coomassie blue stained SDS-PAGE with major protein bands of the *Candida* cytoplasmic antigen fraction observed at 55kDa, 35 to 45kDa region, 30kDa and 20kDa.
Figure 2 shows a single coomassie blue band of 48kDa corresponding to the expected size of the enolase antigen.
Figure 3 shows a coomassie blue stained gel of the clarified cell wall antigen preparation. A broad smear of stain can be seen ranging in size from 90kDa to 200kDa
Figure 4 shows a number of sera screened against the *Candida* cytoplasmic antigen preparation.
Figure 5 shows antibody reactivity to the three Candida antigens - cytoplasmic, cell wall and immunodominant antigens, using negative control sera.
Figure 6 shows antibody reactivity to the three Candida antigens - cytoplasmic, cell wall and immunodominant antigens, using sera from patients with superficial candidiasis.
Figure 7 shows antibody reactivity to the three Candida antigens - cytoplasmic, cell wall and immunodominant antigens, using sera from patients with systemic candidiasis.
Figure 8 shows the error bar of the Applicant antigen test values in the different blood culture patients (95%CI).
Figure 9 shows an error plot of the mean *Candida* antibody values measured by the Applicant antigen test in both the blood culture positive and negative groups of patients (95% confidence interval).
Figure 10 shows an error bar graph of the Applicant antigen test data for invasive candidiasis and healthy controls.

### ABBREVIATIONS USED

- EDTA: Ethylenediaminetetraacetic acid
- EIA: Enzyme immunoassay
- ELISA: Enzyme-linked immunosorbent assay
- RIA: Radioimmunoassay
- BSA: Bovine serum albumin
- DMSO: Dimethyl sulfoxide
- β-Me: β-mercaptoethanol
- TMB: 3,3',5,5'-tetramethyl-benzidine

### DETAILED DESCRIPTION OF THE PREFERRED EMBODIMENTS

The practice of the present invention employs, unless otherwise indicated, conventional molecular biology, cellular biology, and immunoassay techniques within the skill of the art. Such techniques are well known to the skilled worker, and are explained fully in the literature. *See,* e.g., Harlow and Lane, "Antibodies: A Laboratory Manual" (1988); Maniatis, Fritsch & Sambrook, "Molecular Cloning: A Laboratory Manual" (1982); "Animal Cell Culture" (R.I. Freshney, ed., 1986); "Immobilised Cells and Enzymes" (IRL Press, 1986); B. Perbal, "A Practical Guide to Molecular Cloning" (1984); Sambrook, et al., "Molecular Cloning: a Laboratory Manual" (1989) and Ausubel, F. et al., 1989-1999, "Current Protocols in Molecular Biology" (Green Publishing, New York).

In describing the present invention, the following terminology is used in accordance with the definitions set out below.

As used herein, a "biological sample" refers to a sample of tissue or fluid isolated from a individual, including but not limited to bone marrow, plasma, serum, spinal fluid, lymph fluid, the external sections of the skin, respiratory, intestinal, and genitourinary tracts, tears, saliva, milk, blood; both whole blood and anti-coagulated whole blood, blood cells, tumours, organs, and also includes samples of *in vivo* cell culture constituents, including but not limited to conditioned medium resulting from the growth of cells in cell culture medium, putatively *Candida* infected cells, recombinant cells, and cell components.

"Human tissue" is an aggregate of human cells which may constitute a solid mass. This term also encompasses a suspension of human cells, such as blood cells, or a human cell line.

For the purposes of this specification it will be clearly understood that the word "comprising" means "including but not limited to", and that the word "comprises" has a corresponding meaning.

It will be clearly understood that, although a number of prior art publications are referred to herein, this reference does not constitute an admission that any of these documents forms part of the common general knowledge in the art, in Australia or in any other country.

Persons skilled in the art will appreciate that any number of different immunoassays may be used in the present invention. For example, the *Candida* antigens disclosed herein may be used in antibody capture assays, antigen capture assays, wherein the antigen/antibody complex forms a "special" class of antigen or two-antibody sandwich assays.

### TECHNIQUES USED FOR ANTIGEN PREPARATION

The term "*Candida* antigen" as used here means any one of the three separate types of *Candida* antigen utilised in the present invention, namely, cell wall antigen (including mannose), total cytoplasmic antigen (mannose depleted) or purified immunodominant antigen (enolase). Use of the term "*Candida* antigens" means that all three antigens were involved or could be utilised. A number of techniques may be used to prepare the *Candida* antigens including biochemical extraction, column chromatography, Gel fractionation, gene cloning, differential precipitation, filtration, dialysis or centrifugation; however, the preferred techniques are those disclosed herein. Briefly, these techniques involve either mechanical, chemical or enzymatic lysis of *Candida* cells, followed by separation of insoluble cell walls from soluble cytoplasmic fraction by centrifugation, filtration and dialysis. Chemical treatment of cell wall fraction to release cell wall antigens followed by centrifugation and dialysis. Filtration and organic extraction of soluble cytoplasmic cell extract. Separation of mannoproteins by ConA affinity chromatography. Purification of the immunodominant enolase antigen from the soluble cytoplasmic extract by anion and cation affinity chromatography. It will be appreciated by those skilled in the art that other techniques, or modifications or variations of the above techniques, may be adopted without adversely affecting the spirit of the present invention.

### TECHNIQUES USED FOR ANTIBODY PREPARATION AND LABELLING

Antiserum to the *Candida* antigens disclosed herein may be produced in a host animal such as rabbit or sheep. The serum fraction containing the antibody may be isolated by standard techniques. This antiserum may be employed in several of the embodiments of the invention hereinafter set forth, or a more sensitive and specific antibody might be obtained by further purification of the serum by electrophoresis, high-speed centrifugation or the like. Ultimately, large quantities of highly specific monoclonal antibody may be produced by means of the hybrid-myeloma techniques by methods known to those skilled in the art.

Certain embodiments of the present invention employ antibody to the *Candida* antigens immobilised on cellulose, agarose, sephadex or glass beads or other similar inert surfaces such as metal, plastic or ceramic which do not interfere with subsequent reaction. Adsorption, Br-CN activation or other techniques known in the art may be employed to immobilise the antibody.

Other embodiments of the present invention employ the antibody to the *Candida* antigens conjugated to a chromophoric (highly coloured) molecule, an enzochromic (an enzyme which produces colour upon addition of reagents) molecule, fluorochromic (fluorescent) molecule or a luminogenic (luminescent) molecule.

The conjugate of antibody with enzyme is made using techniques known in the prior art. (For references, see Avrameas, S. and Uriel, J., in Comptes Rendus Hebdomadaires des Seances de l'Academie des Sciences, vol. 262, p. 2543, (1966); Nakane, P.K. and Pierce, G.B., in Journal of Histochemistry and Cytochemistry, vol. 14, p. 929, (1966); Nakane, P.K., in Methods in Enzymology, vol. 37, p. 133, (1975)).

Chromophoric molecules that may be used are 2,3-dinitrobenzene (DNB) salts, dinitrophenol (DNP) and methyl and butyl orange. Other suitable chromophoric agents are well known in the art. Enzochromic molecules that may be conjugated with the antibody are enzymes that give colour with appropriate reagents. Examples are alkaline phosphatase (ALP) which develops colour with nitrophenyl phosphate (NPP), glucose oxidase with glucose, and D-galactopyranoside. These and other examples are well known in the art. Examples of fluorogenic agents are 2,4-dinitrofluorobenzene and "pipsyl" derivatives. Luminogenic molecules may be conjugated to antibodies by the method of Branchini, et al. (Biochem. Biophys. Res. Commun. 97, 334 [1980]). The term "chromophoric" hereinafter is intended to include "enzochromic", "fluorochromic" and "luminogenic" molecules as well.

Certain embodiments of the invention also utilise *Candida* antibody tagged with a radioactive clement. I¹²⁵ conjugated by means of the chloramine-T procedure is a common example, but other methods known in the art may also be employed.

### TECHNIQUES USED FOR ANTIGEN IMMOBILISATION AND LABELLING

Antigen molecules may be immobilised on a solid carrier by a variety of methods known in the art, including covalent coupling, direct adsorption, physical entrapment and attachment to a protein-coated surface. For references describing the methodology, see Silman, I. H. and Katchalski, E. in Annual Review of Biochemistry, Vol. 35, p. 873 (1966); Melrose, G. J. H., in Review of Pure and Applied Chemistry, Vol. 21, p. 83, (1971); and Cuatrecasas, P. and Anfinsen, C. B., in Methods in Enzymology, Vol. 22, (1971).

Lai et al. (German OS No. 2,539,657, U.S. Pat. No. 4,066,512) discloses a method of attachment to a protein-coated surface. In this method, the internal and external surfaces of a microporous membrane are first coated with a water-insoluble protein such as zein, collagen, fibrinogen, keratin, glutelin, polyisoleucine, polytryptophan, polyphenylalanine, polytyrosine, or copolymers of leucine with p-amino phenylalanine. Such a coating renders the membrane capable of immobilising a wide variety of biologically active proteins including enzymes, antigens, and antibodies. A microporous structure is defined as one having more than 50% of its total volume in the form of pores ranging in size from 25 nanometres to 25 micrometers, preferably from 25 nanometres to 14 micrometers. A pore size range from 25 nanometres to 5 micrometers is employed in most applications herein. Uncoated microporous membranes have as much as 70 to 75% of their volume as pore space. The pores permit liquid flow through the membrane. After being coated by zein, for example, the pore space is reduced 5 to 10% with the result that the structure retains its essential properties of having a high proportion of its volume as pore space and permitting liquid flow through the pores. The structure has a large surface area in contact with any solution contained within the pores.

Such a coated membrane, having immobilised antigen or antibody, provides a compact, easy to manipulate carrier for the immobilised antigen or antibody. Its integral structure permits removal of bound from unbound components by simple mechanical means.

Non-specific binding may be minimised by interposing a second stage immobilisation step, in which an immunochemically neutral protein is immobilised to the filter. Immobilisation therefore occurs in two stages according to a preferred embodiment of the invention: a first stage in which the desired immunochemical component is immobilised, and a second stage, following the completion of the first, in which an immunochemically neutral protein such as fetal calf serum or bovine gamma globulin is next immobilised. The term immunochemically neutral is defined in terms of the specific components of the assay. Any protein, which does not combine immunochemically with a component of the assay or with one of the reagents, is considered immunochemically neutral, even though such protein might be immunochemically reactive in another system.

Where the substance to be detected is an antibody, the immunochemically reactive moiety of the conjugate must be an antibody capable of binding immunochemically with the antibody to be tested. Such antibodies may be obtained by immunising an animal with the antibody or immunoglobulin fraction of serum from the animal in which the antibody to be tested originated. For example, where the antibody to be tested is a human antibody, a goat antibody against human antibody is obtained from the serum of a goat immunised against human immunoglobulin (antibody). The enzyme moiety maybe any enzyme capable of catalysing a reaction which can be detected by any method known to those skilled in the art, and which retains its activity after conjugation with antibody. Horseradish peroxidase is preferred because of its convenience and suitability to a wide range of applications. It is well known that the enzyme catalyses the oxidations of a variety of organic compounds in the presence of hydrogen peroxide. Many such organic substrates are chromogenic, ie. undergo a colour change upon oxidation.

It has been found in the present invention that the purity of the enzyme preparation used in the formation of conjugate has an effect on the degree of non-specific binding. The greater the purity of the enzyme preparation, the less the non-specific binding. In part, the reduction is made possible because, the total amount of conjugate protein required is reduced as the specific activity of the enzyme is increased. The opportunity for non-specific binding is therefore reduced as well. In the preferred embodiment, the use of a highly purified peroxidase preparation has been found to significantly reduce the amount of colour reaction observed in control samples as compared with known positives. TECHNIQUES USED FOR *CANDIDA* ANTIBODY DETECTION

### Antibody Capture Technique

A *Candida* antigen prepared by the techniques disclosed herein is immobilised, preferably on an inert surface such as PVC, paper or a similar bibulous mat. The immobilised *Candida* antigen is then put into contact with a sample suspected of containing *Candida* antibody. In the case of aqueous samples such as blood or urine, the solution is buffered and ionic salts may be present at optimum concentration for *Candida* antibody-*Candida* antigen interaction. TRIS or borate buffered phosphate at pH 7.5 to 9.0 and ionic strength about 0.010 to 0.5, for example, are suitable buffering agents and ionic salts. The inert surface with *Candida* antigen or *Candida* antigen-*Candida* antibody complex thereon is next put into contact with antibody to *Candida* antigen conjugated to a chromophoric molecule. Preferably the *Candida* antigen is in solution buffered at pH from about 7.5 to 9.0 and ionic concentration equivalent to about 0.01M to about 0.1M NaCl. After careful rinsing under water or with suitable surfactants such as Tween 20 to remove excess coloured antibody, the inert surface is inspected for colour, fluorescence or luminescence directly or after addition of colour-developing agents. Colour on the inert surface indicates interaction between immobilised *Candida* antigen-*Candida* antibody complex in solution. A control may be run for colour comparison.

This technique may be adapted to clinical use by employing *Candida* antigens tagged with radioactive elements and observing either depletion of activity in solution or uptake on solid support of radioactivity. This embodiment is highly sensitive and rapid and suitable for large numbers of samples.

### Enzyme-Linked Immunoassay-ELISA

A solution comprising *Candida* antibody conjugated to enzyme which forms colour with developing reagents and buffer and ionic salts suitable for reaction between *Candida* antigen and the *Candida* antibody is put into contact and allowed to react with *Candida* antigen immobilised, preferably, on an inert surface such as PVC, paper strip or glass bead. The amount of enzochromic conjugated *Candida* antibody is sufficient to saturate about 50% of the reactive sites on the immobilized antibody. The inert surface with antibody-*Candida* antigen enzyme complex is put into contact with buffered sample suspected of containing *Candida,* said sample having an unknown amount of *Candida* antibody. The colour of the resultant immobilised antibody-*Candida* antigen-enzyme complex on the strip after colour developing reagents are added is observed in comparison to a control strip which has not been treated with sample containing *Candida* antibody. Dilution in colour on inert surface treated with sample means presence of *Candida* antibody in the unknown sample.

This method may he adapted for clinical use by contacting samples and immobilised enzyme, preferably in tubes which may be centrifuged and watching developing colour spectrophotometrically. This embodiment is very sensitive and rapid.

### Radialimmunodiffusion-Precipitin Reaction

One of the *Candida* antigens is suspended in a softened gelatinous medium such as agar or agarose along with buffers and salts to maintain pH between about 6.0 to 9.0 and ionic strength between about 0.01M to 0.5M for optimal antigen-antibody interaction. The suspending medium of U.S. Pat. No. 4,259,207 is a suitable example. The mixture is spread out to harden on a test plate or, preferably, poured into a disc-shaped container such as an Octolony plate. A small amount of sample is placed on the solidified gel, preferably in a centre well and the plate or disc is allowed to stand preferably covered for a period of hours. Diffusion of sample into the surrounding area occurs during this period. If the *Candida* antibody is present, it reacts with the embedded *Candida* antigen and causes an opaque area in a radial pattern about the point of application of sample. A control can be run for comparison. Calibration of an amount of *Candida* antibody in the sample, if desired, can be obtained by controlling temperature, time and size of sample and comparing the resultant size of radial area with one of known concentration.

### Radioimmunoassay

A *Candida* antigen of the present invention is immobilised on an inert surface such as glass beads in a separation column. A portion of *Candida* antigen is conjugated to a radioactive element, preferably I¹²⁵ and allowed to react with the immobilised *Candida* antigen in an amount sufficient to, saturate 50% of the binding sites. The immobilised *Candida* antigen-enzyme complex is put into contact with a sample suspected of containing *Candida* antibody, the sample being buffered between pH 6-9 and containing total ionic salts about 0.05 to 0.5M for optimal reaction conditions for formation of *Candida* antigen-antibody complex. The *Candida* antibody is eluted from the antigen and the eluant is measured for radioactivity. Loss of activity compared to a control indicates *Candida* antibody in the sample.

### Haemagglutination

*Candida* antibody may be assayed through standard haemagglutination techniques with *Candida* antigen to antibody used as sensitising agent.

It is to be understood that methods described hereinabove for assay of *Candida* antibody employing coloured reagents have been presented most specifically for application where neither trained personnel nor sophisticated instruments are available. These methods, however, may be adapted for use in a clinical setting where large numbers of samples are to be assayed by substituting radioactive elements for chromogenic conjugated molecules.

It is also to be understood that the term "colour" is not to be interpreted as being limited to the narrow visible range of the electromagnetic spectrum, but is meant to include wavelengths which may be measured by standard spectrophotographic instruments such as spectrophotometers and absorption and emission colourimeters in both the uv and the ir range.

Although it is contemplated that the methods of the present invention are to be applies to biological fluids themselves, the sensitivity and specificity of the method can be improved by culture of the fluids preferably on medium selective for *Candida* prior to testing.

Sensitivity may also be improved by preliminary treatment of biological samples with lysing agents such as isotonic solution, sound, or lysozyme to release *Candida* antibody into the extracellular environment. U.S. Pat. No. 4,166,765, for example, discloses suitable lysing procedures for biological samples containing bacteria. Any lysing agent may be employed which does not interfere with subsequent enzyme activity.

### ASSAYS EMBODIED IN KIT FORM

The diagnostic method and means of the present invention may be embodied in the form of a kit for use by individuals for self-diagnosis of *Candida* in the privacy of their homes.

The kit comprises a means for sample collection, the *Candida* antigen to *Candida* antibody and a means for detecting reaction between sample and *Candida* antigen.

In embodiments adapted for clinical use, electrophoretic separation techniques such as isoelectric focusing or zone electrophoresis which are based on differences of both size and charge distribution between products and reactants may likewise be used to separate products from reactants. Products separated electrophoretically may be detected by characteristic locations compared to standards or may be identified by colour or immunochemically. Resinous beads of charged surfaces may also be used to separate products and reactants.

The means for detecting reaction in the case of immunoassay in a preferred embodiment of the invention is a gelatinous medium in which the *Candida* antigen to antibody is suspended. The gelatinous medium is in a transparent glass or plastic container and comprises buffer and ionic salts for optimal conditions for formation of the *Candida* antigen-antibody complex. Reaction is noted as a transparent area radiating from the central point at which the sample is applied.

The means for detecting reaction in another preferred embodiment comprising immunoassay is the *Candida* antigen to *Candida* antibody conjugated to a chromophore in a sealed, sterile packet along with buffer and ionic salts. For assay, the contents of the packet are diluted with water in a marked tube supplied in the kit. Included also in this embodiment is the antigen to *Candida* antibody immobilised on an inert surface. For assay, the inert surface with immobilised *Candida* antigen is put into contact with sample and then with the solution of chromophore-conjugated *Candida* anti-IgA antibody, protein A or protein G. The inert surface is inspected for colour, which indicates *Candida.*

In a particularly preferred embodiment, the kit of the present invention is provided in the form of an immunochromatographic test strip device. There are many patents that cover a number of technologies, formats, reagents and materials that may be of great value in the development and production of immunochromatographic test strip devices. For example, US Patent No. 5,075,078, International Patent Application No. WO95/16207, US Patent No. 5,654,162 and European Patent No. 0810436A1. The assay methods used with the devices disclosed in these patents are essentially the same. A ligand specific for the analyte (normally, but not necessarily an antibody [Ab]) is immobilised to a membrane such as nitrocellulose. The detector reagent, typically an antibody coupled to latex or colloidal metal, is deposited (but remains unbound) into the conjugate pad. When sample (urine, plasma, whole blood, etc.) is added to the sample pad, it rapidly wets through to the conjugate pad and the detector reagent is solubilised. The detector reagent begins to move with the sample flow front up the membrane strip. Analyte that is present in the sample will be bound by the antibody that is coupled to the detector reagent. As the sample passes over the zone to which the capture reagent has been immobilised, the analyte detector reagent complex is trapped. Colour develops in proportion to the amount of analyte present in the sample.

In the present case, while the above principles are the same, rather than detecting analyte per *se,* the immunochromatographic test strip device would detect antibody. In such situations, it would be the antigen(s) disclosed herein which would immobilised onto membranes, sample pads, reagent pads and other porous media rather than antibody. There are a wealth of information regarding the development of such devices including methods of binding antigen/antibodies to nitrocellulose and the like and detecting such bound material. See for example, Towbin et al. 1979, Proc. Natl. Acad. Sci. USA 76:4350, the entirety of which is included herein by reference.

Although the invention has been described with reference to presently preferred embodiments, it should he understood that various modifications can be made without departing from the spirit of the invention. Moreover, the following examples are offered by way of illustration only and are not intended to limit the invention in any manner. All patent and literature references cited herein are expressly incorporated.

### EXAMPLE 1 REPARATION OF CANDIDA ANTIGEN

The following three types of *Candida* antigen were prepared:
1). Cell wall antigen (including mannose);
2). Total cytoplasmic antigen (mannose depleted); and
3). Purified immunodominant antigen (enolase).

A clinical isolate of the *Candida albicans,* was obtained from a patient with vaginal thrush. The identity of the *Candida* species was confirmed with the use of an API 20C Auxonagram strip (API System S.A., France). The *C. albicans* isolate was designated KEMHS.

200ml YEPD culture medium (1% yeast extract, 2% peptone, 2% D-glucose) was inoculated with the isolate as a starter culture and incubated for 24h at 30°C with aeration. The starter culture was then used to inoculate a 10L YEPD culture incubated under similar conditions in a 23L Bio-Flo Fermenter IV System (New Brunswick Scientific, Edison, NJ).

The *Candida* culture was harvested from the Bio-Flo fermenter system and separated from culture medium with the use of a Pellicon filtration cassette (Millipore, USA). Concentrated cells were separated from residual medium by centrifugation in 500ml centrifuge flasks for 15min at 1,660 x g and 4°C. The supernatant was discarded and the pelleted cells were resuspended in protein extraction buffer (20mM bis-Tris, pH 6.5). The yeast cells were then centrifuged as described previously, resuspended and pooled for further processing.

*Candida* cells were ruptured mechanically with the use of a Dynomill^{®} (WAB, Switzerland). Milling was continued until 99% cell disruption was obtained. The soluble *Candida* cell extracts were collected and dispensed into 50ml centrifuge tubes. The extracts were centrifuged for 12h at 8,517 x g and 4°C to precipitate insoluble cell walls. The supernatants containing the soluble cytoplasmic antigen fraction were recovered and passed through a 0.45µm filter membrane.

The filtrates were then extracted with an equal volume of chilled chloroform. Following centrifugation at 4°C for 15min at 1,036 x g the upper aqueous phase was aspirated and transferred to a dialysis tube. The soluble cytoplasmic protein fractions were dialysed in column binding buffer (20mM Tris/HCl, pH 7.4, 0.5M NaCl, 1mM MnCl₂.4H₂0, 1mM CaCl₂) for 12h in preparation for chromatography.

The soluble cytoplasmic antigen fraction was depleted of contaminating soluble cell wall mannoprotein by Con A-Sepharose chromatography. The dialysed cytoplasmic antigen fraction was filtered through a 0.45µm filter. 50ml of the dialysed extract was applied onto a Con A-Sepharose column (2.6 x 12.5 cm) equilibrated in binding buffer at a flow rate of 4ml/min. The unbound flow-through fraction (non-glycosylated proteins) was collected. Bound mannoproteins were eluted with 0.5M α-methyl mannoside in binding buffer. This step was performed before the next run and to clean the column before storage.

The soluble cytoplasmic antigen fraction was dialysed overnight against 20mM Tris.Cl, pH7.4. An estimate of the quantity of protein in solution was performed using the Bio-Rad^{®} (Bradford) microassay procedure in accordance with the manufacturers instructions. A portion of the cytoplasmic antigen extract was analysed by SDS-PAGE.

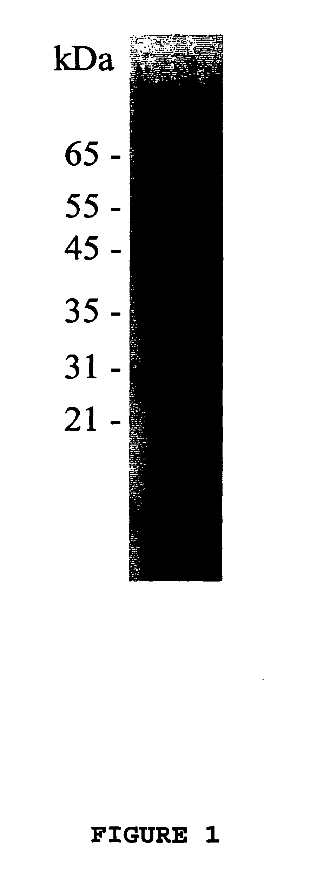

As shown in Figure 1 there was a number of major protein bands observed which varyed in size from approx 20kDa up to approx 60kDa in size. The major staining bands being at 55kDa, four bands in the 35 to 45kDa region, 30kDa and 20kDa. This was in stark contrast to the large number of Coomassie blue staining bands in the original crude lysate prior to organic extraction and Con A-Sepharose chromatography.

Purification of the enolase antigen was conducted in the same fashion as the soluble *Candida* cytoplasmic antigen except that it was not subjected to Con A-Sepharose chromatography. Instead, following dialysis and filtering through a 0.20µm syringe filter (cellulose acetate), the filtered extracts were applied to a Pharmacia Biotech XK 50/20 chromatography column packed with Pharmacia Biotech Source 15Q quaternary ammonium anion exchanger (Pharmacia LKB, Uppsala, Sweden). The column was equilibrated prior to chromatography with column binding buffer 'A' (20mM bis-Tris, pH 6.5). Anion exchange chromatography of the crude extract was controlled and recorded using the Bio-Rad^{®} Econo^{®} system (Bio-Rad Laboratories, USA). Bound protein was eluted from the column with a salt gradient of buffer 'B' (1M NaCl in buffer 'A', pH 6.5). The recovered fractionates proteins were analysed by an enzyme activity assay.

The active enzyme enolase hydrolyses D(+)2-phosphoglyceric acid (PGA) to phosphoenolpyruvate (PEP). The production of PEP can be monitored by spectrophotometry at 240nm. 20µl of protein solution was combined with 1ml of enolase substrate solution (50mM Tris-HCl pH 7.4, 2.7mM magnesium acetate, 1.0mM EDTA, 1.2mM D(+)2-phosphoglyceric acid) in a quartz cuvette and the change of absorbance recorded at 1min intervals. The specific activity was defined as the conversion of 1µmol of PGA to PEP per min per mg protein. An estimate of the quantity of protein in solution was performed using the Bio-Rad^{®} (Bradford) microassay procedure.

Eluate fractions containing enolase activity were selected and dialysed for 12h at 25°C in hpH₂O. The dialysed fractions were recovered and filtered through a 0.20µm syringe filter. The filtrate was concentrated tenfold by evaporation under vacuum for 5h. The concentrated samples were dialysed with binding buffer 'A' (10mM sodium acetate, pH 4.7) immediately prior to application to a Pharmacia Biotech Mono S HR10/10 chromatography column packed with methyl sulphonate cation exchanger (Pharmacia LKB, Uppsala, Sweden). Cation exchange chromatography was performed using the Bio-Rad^{®} Biologic system. Bound protein fractions were eluted from the column with a salt gradient of buffer 'B' (1M NaCl in buffer 'A', pH 4.7). Fractions containing enolase activity were identified by the enzyme activity assay described above.

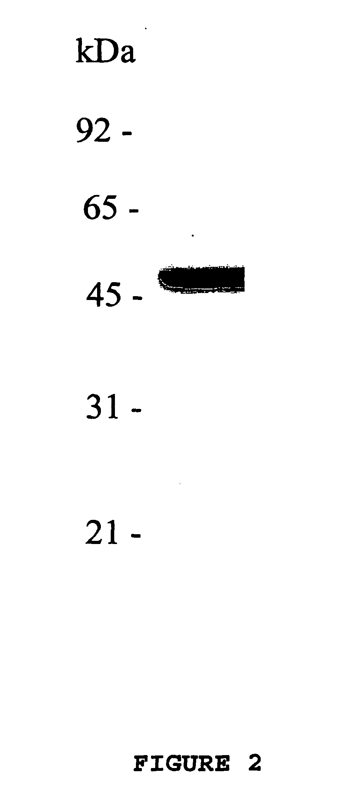

Figure 2 shows a single Coomassie blue band of 48kDa corresponding to the expected size of the enolase antigen. The identification of the 48kDa antigen as the glycolytic enzyme enolase was confirmed by an enolase activity assay.

Purification of the cell wall antigen was conducted as follows: the precipitated insoluble cell walls were collected following centrifugation as described above. The cell walls were washed with hpH₂O then collected by centrifugation at 6,000 rpm. This step was repeated three times or until the supernatant was no longer cloudy. This ensured any residual soluble cytoplasmic antigen was removed from the cell wall preparation. The washed cell wall pellet was then resuspend in 10mM Phosphate buffer pH7.4 containing 1% v/v β-Me and incubated for 30min at 37°C in a shaker to solubilise the cell wall antigens. The sample was then centrifuged for 5min at 8,000 rpm and the pellet was then discarded. The supernatant was transferred into a fresh tube and recentrifuged (5min at 8,000 rpm). The supernatant containing the solubilised cell wall antigen was then dialysed in hpH₂O for 48h at 4°C (four changes of water), or until no odour was detected. Following dialysis the sample was centrifuged three times 5min at 8,000 rpm to remove any residual particular matter.

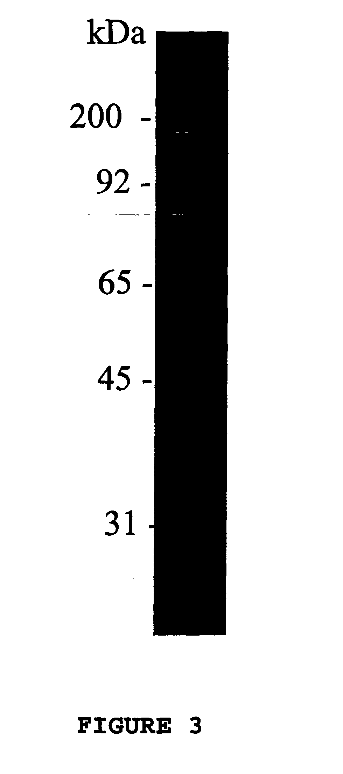

Following clarification the cell wall antigen preparation was analysed by SDS-PAGE. The resulting Coomassie blue stained gel is presented in Figure 3. A broad smear of stain is seen ranging in size from 90 kDa to 200 kDa. The lack of discrete protein bands is typical of mannoproteins, where differences in the number of mannose groups added to the protein base results in a variety of molecular weights.

### EXAMPLE 2 ENZYME LINKED IMMUNOSORBENT ASSAYS (ELISAS)

A serum panel was collected from 1998 to 2000 from various patients with *Candida* infections. Negative control (Control) sera (n=20) were obtained from the Red Cross Blood Bank, Perth, Australia and was obtained from healthy males in the 19 to 25 year age group. Sera (n=13) from patients with recurrent vulvo vaginal candidiasis (VVC) were obtained from King Edward Memorial Hospital, Perth, Australia. Sera (n=108) from patients with oral candidiasis were obtained from Clinipath Ltd and the UWA Dental School, Perth, Australia. Sera (n=39) from patients (n=28) with systemic candidiasis were obtained from Princess Margaret Hospital, Perth, Australia and Prince of Wales Hospital, Sydney, Australia.

In the case of patients with oral and vaginal *Candida* infection, confirmation of infection was made by physical examination and by culture of *Candida* organisms from the relevant body site. In the case of patients with systemic infection, confirmation of infection was through positive blood culture or biopsy. In all cases the immune status of the patient was unknown.

Sera from patients with either superficial or systemic candidiasis were screened by ELISA using trays coated with the *Candida* cytoplasmic antigen. The protein content of each antigen preparation was determined using a commercial assay (BioRad) with BSA as a standard. A series of ELISAs were performed to determine the optimal coating concentration for each antigen (data not shown). The optimal coating concentration being that which gave the greatest discrimination between a positive and a negative control serum. For each antigen the optimum coating concentration was determined to be 2µg/ml.

A 96 well C8 strip microtitre plate (Greiner GmbH, Germany), was coated with either *Candida* cell wall antigen, cytoplasmic antigen, or purified enolase antigen as prepared in Example 1. 50µl of a 2.0µg/ml solution of the antigen was diluted in coating buffer (0.1M NaHCO₃, pH 9.3) and added to individual wells. The plates were incubated for 12h at 4°C then equilibrated to ambient temperature. After equilibrating the plates to ambient temperature, coating solution was decanted and the plate tapped dried. Plates were inverted on paper towel to drain. Alternatively excess coating solution was aspirated by the automated plate washer (Dynatech Laboratories, Chantilly VA, USA). It was important not to wash the plate at this stage.

A volume of 300µl of blocking solution (PBS pH 7.3, 2% (w/v) BSA (ICN, Australia), 0.01% (w/v) Tween 20), was applied to each well and incubated at 25°C for 90min. Blocking solution was decanted and the plate tapped dried. Plates were inverted on paper towel to drain and tapped dried for a second time. At this stage plates were either used immediately, or dried for storage. Plates to be dried were placed inverted in a sealable container such as a plastic food container with a number of silica gel desiccant sachets for 48h. The inclusion of approximately 20 small desiccant sachets was adequate for the drying of 6 coated ELISA micro-well trays. Dried plates were sealed into heat-sealed packets with a single desiccant sachet and labelled. Plates were stored at 4°C until required. Packets containing plates were equilibrated to ambient temperature before opening.

Human test sera diluted 1/100 in blocking solution was dispensed into wells in 50µl aliquot's and incubated at 37°C for 30min. The primary antibody solution was aspirated and wells were washed six times in PBS-Tween 20. The plates were inverted on paper towels and allowed to drain for 10min. The plates were then tapped dried.

A volume of 100µl of a horseradish peroxidase anti-human IgG conjugate diluted 1/10,000 in blocking solution was dispensed to each well. Secondary antibody solution was incubated at 37°C for 30min. The secondary antibody solution was aspirated and wells were washed six times in PBS-Tween 20. Plates were inverted on paper towel to drain for 10min and then tapped dried. Plates were inverted on paper towel for a second time and allowed to drain for 5min. Plates were then tapped dried. Particular care was employed to ensure that all traces of secondary conjugate solution was removed as residual conjugate was established as the major factor responsible for disparity of results (Dynatech Laboratories Inc, USA).

A volume of 100µl of TMB liquid substrate solution was dispensed into each well and developed at 25°C for 10min. The reaction was terminated with the addition of 100µl of 1M phosphoric acid or 1M H₂SO₄. The absorbance values for each well were measured at 450nm, reference 620nm with a MRX automated plate reader.

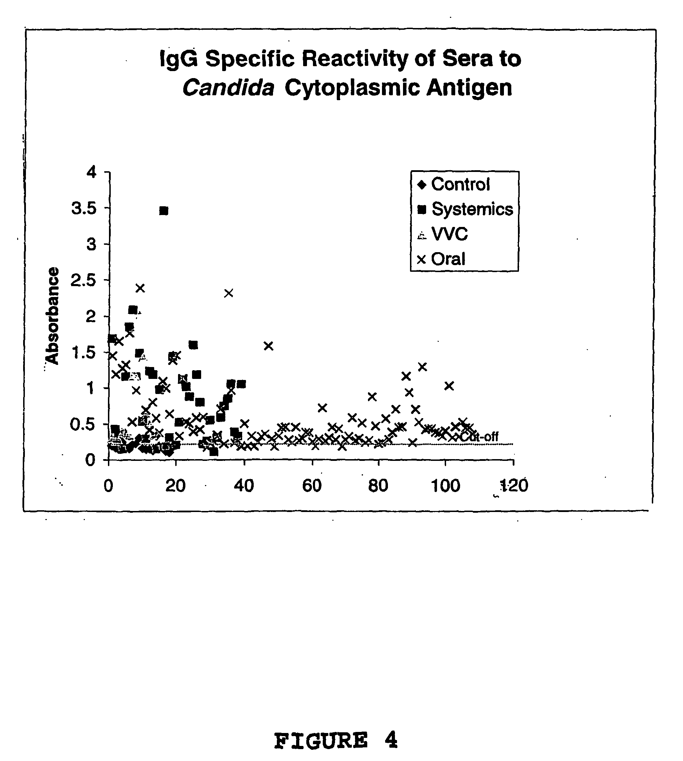

Each immunoassay was performed in triplicate and the mean value of absorbance was used. The absorbances are shown as a Scatter diagram in Figure 4. Three groups of patients with *Candida* infections were analysed. The first group were patients with systemic candidiasis (Systemics), the second group had oral candidiasis (Oral) and the third group had vulvovaginal candidiasis (VVC). Blood bank sera (Control) from males in the 19 to 25 year age group, who were at low risk of having an undetected or subclinical *Candida* infection were used as a control. The cut-off absorbance (OD₄₅₀ = 0.22) was the mean value of the negative control sera. From these data the cytoplasmic antigen ELISA had a sensitivity of 89% and a specificity of 95%. This is higher than that reported for other *Candida* serological tests (Zoller et al., 1991. J. Clin. Micro. 29:1860-1867).

To further increase the sensitivity of the *Candida* ELISA multiple antigens were used. These were the cell wall, cytoplasmic and native enolase (described above).

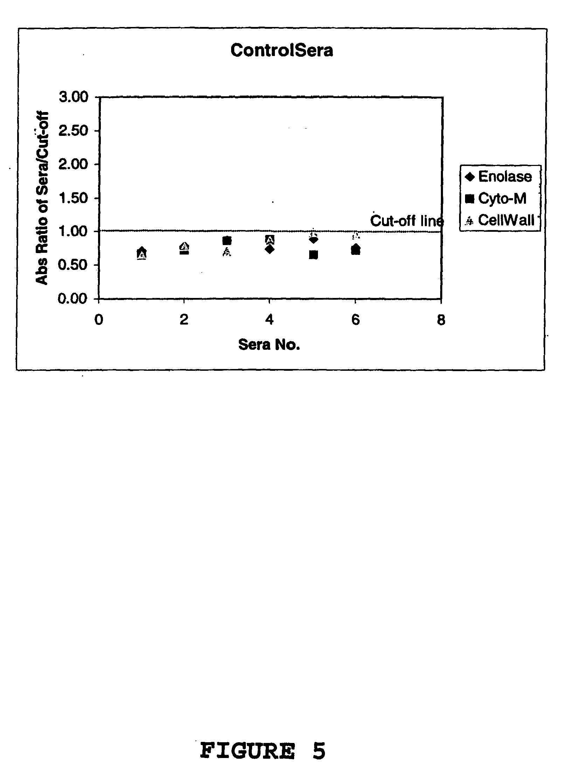

The use of multiple antigens increased the sensitivity of the *Candida* ELISA. It also provided greater discrimination between superficial and systemic infection. Six negative control sera (serum obtained from healthy males in the 19 to 25 year age group) were used in ELISAs with microtitre tray wells individually coated with the three *Candida* antigens. For each serum the antibody titre to each of the three antigens was below that of the cut-off line (Figure 5). This line is the cut off value assigned based on a comparison of the average antibody titres of sera from control patients versus those of candidiasis patients. The value plotted on the y-axis of the graph is the ratio of the cut-off absorbance divided into the absorbance of the test serum.

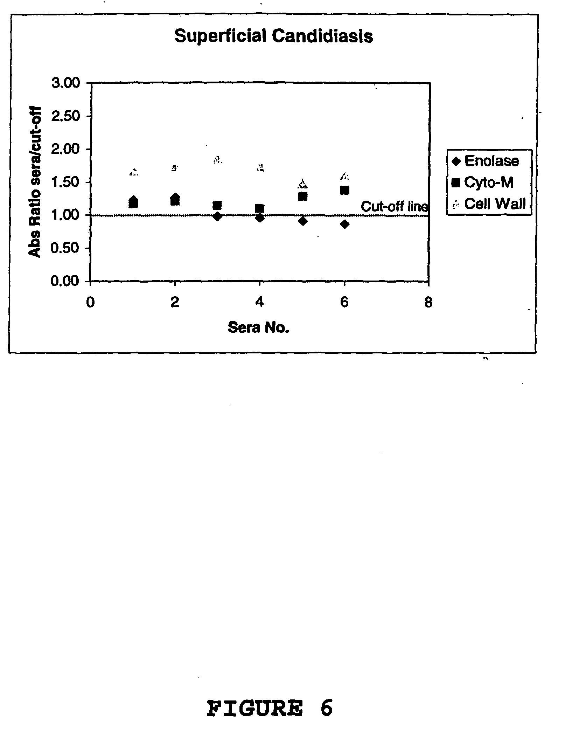

Serum obtained from 6 patients with superficial candidiasis was then reacted in the ELISA. Again the absorbance value of each serum was divided by the absorbance of the cut-off (Figure 6). The characteristic antibody response of the sera from patients with superficial candidiasis was a high titre against the cell wall antigen preparation (1.5 to 2 times the cut-off value). The antibody reactivity to the complete cytoplasmic antigen preparation was positive in most cases (1 to 1.5 times the cut-off). In contrast the antibody titre to the enolase antigen was below or equal to that of the cut-off. There is a correlation between the antibody titre to the internal *Candida* antigens (cytoplasmic and enolase) and the severity of the superficial infection (data not shown). However, the severity of the infection in the six patients analysed was not known.

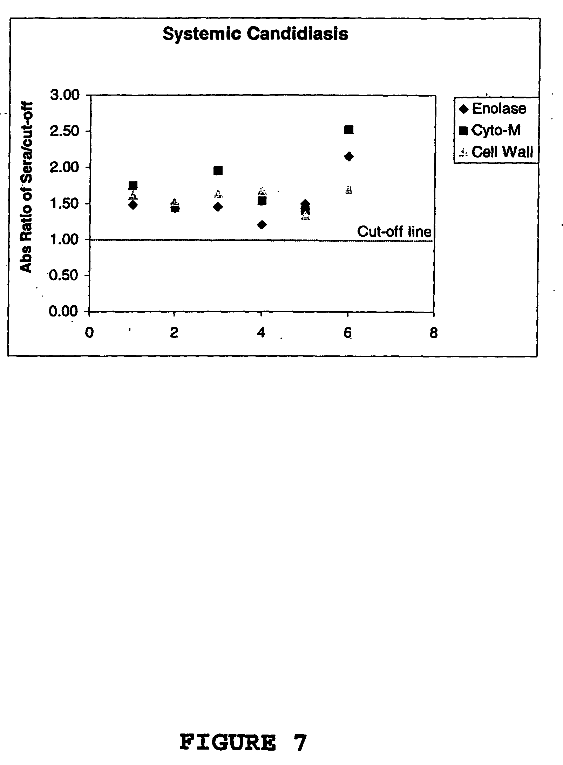

Six sera taken from patients with systemic candidiasis (confirmed by positive blood culture) were analysed by ELISA. The results are presented in Figure 7. In the case of the patients with systemic candidiasis the antibody response to the cell wall antigen preparation was positive (1.5 to 2 times the cut-off value). Also, the antibody titres to the internal *Candida* antigens (cytoplasmic and enolase) were also positive (1.5 to 2.5 times cut-off value).

### CONCLUSIONS

The *Candida* mannan depleted cytoplasmic antigen preparation disclosed herein can be used to identify patients with *Candida* infections. The sensitivity and specificity using an ELISA with microtitre trays coated with this antigen is greater than that obtained by other *Candida* diagnostic tests. Further, the ELISA assay format disclosed herein is easier to perform, more robust and more rapid than formats used in other available *Candida* diagnostic assays. The ELISA format also has the advantage that it is quantifiable. This enables the patient to be monitored over a period of time and changes in the titre of the antibody response to the *Candida* antigens recorded. The ability of the test to monitor overtime the antibody titre to *Candida* antigens has a prognostic value in terns of measuring the patient's response to antifungal drugs and in the overall survival prospects of the patient. Another advantage of the cytoplasmic antigen preparation is that the method developed to produce the antigen is simpler and more rapid than other available procedures (eg. compare with that of Zoller et al., 1991, *supra*).

### EXAMPLE 3 CLINICAL EVALUATION IN FRANCE

Clinical evaluation of the triple antigen test kit as described in Examples 1 and 2 was undertaken in the Department of Parasitology and Medical Mycology at the University of Grenoble Faculty of Medicine, Grenoble, France using stored sera.

Sera from two groups of patients were analysed: those that were blood culture positive and those that were blood culture negative. When possible, sera were taken before, at the time of and after the first day of positive blood culture to be tested. The blood culture negative group was divided into 3 subgroups: Patients that were colonised with *Candida* and were serology positive, patients that were colonised with *Candida* and were serology negative, and patients that were not colonised with *Candida* and serology negative. The sera were obtained from patients hospitalised between 1998 and 2000.

The triple antigen ELIZA test ("the Applicant antigen test") was performed according to Example 2. The cut-off calibrator sera was obtained by pooling sera taken from males in 19 to 25 age group who had no history of *Candida* infections.

Table 1 shows that the Applicant antigen test was positive in 15 out of 19 patients who had a positive blood culture.

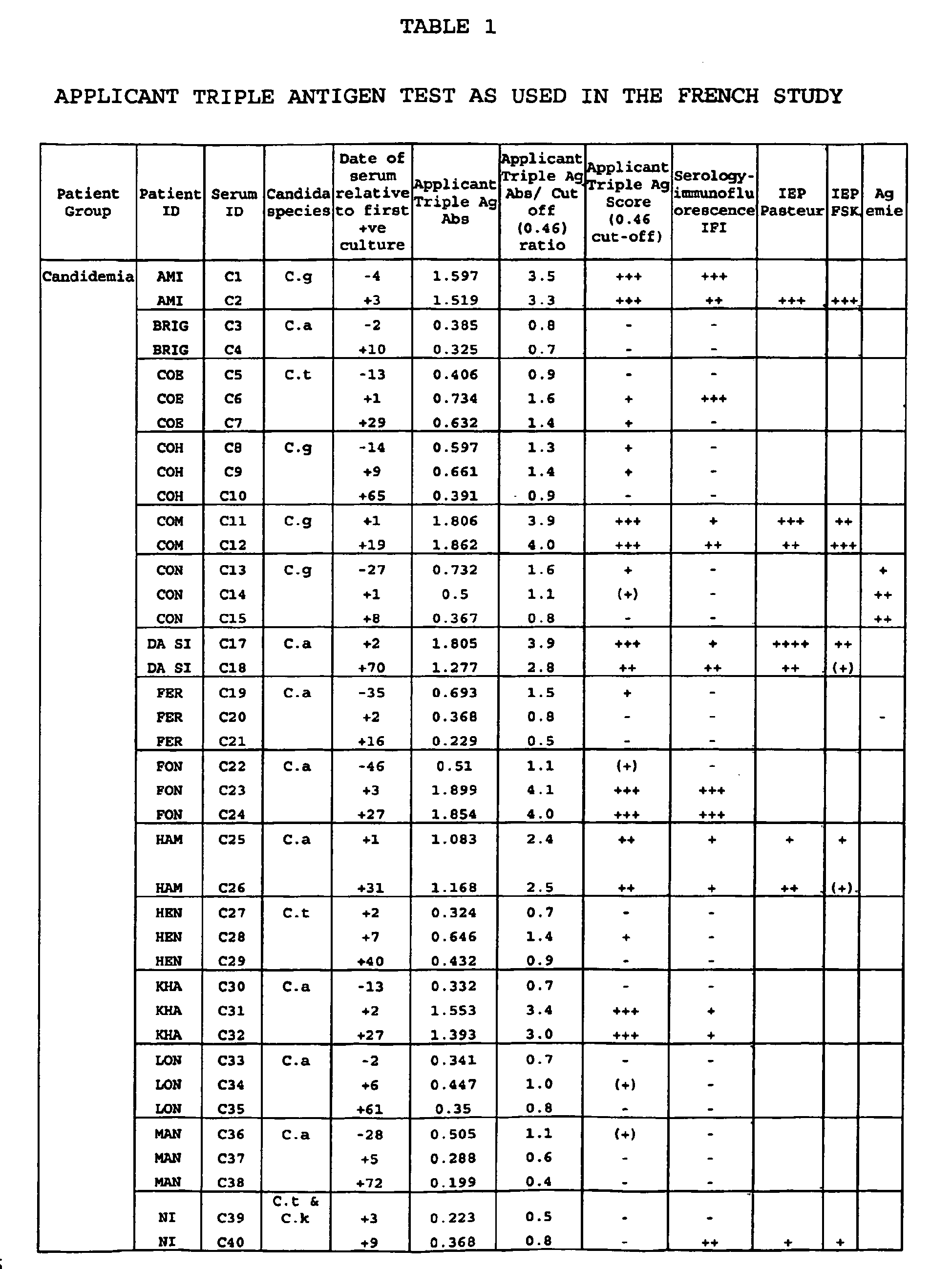

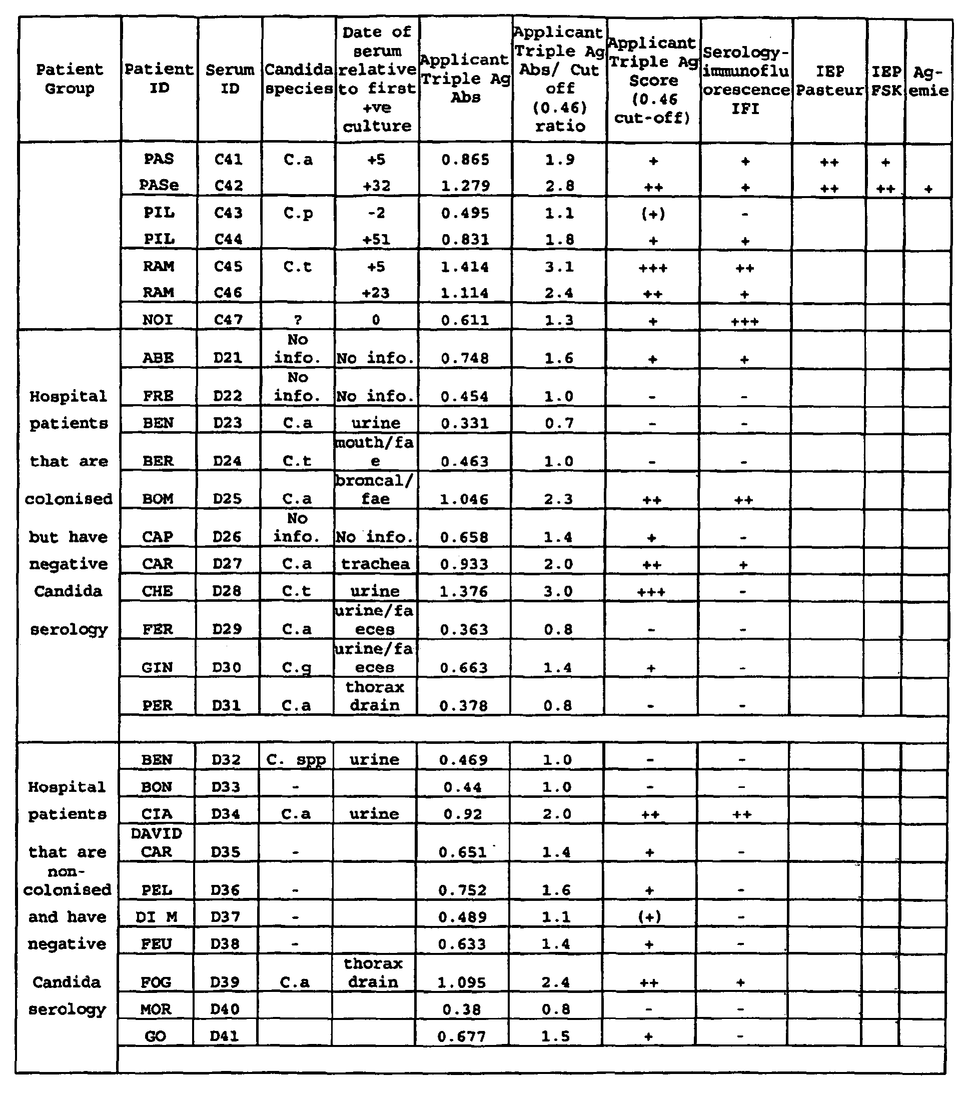

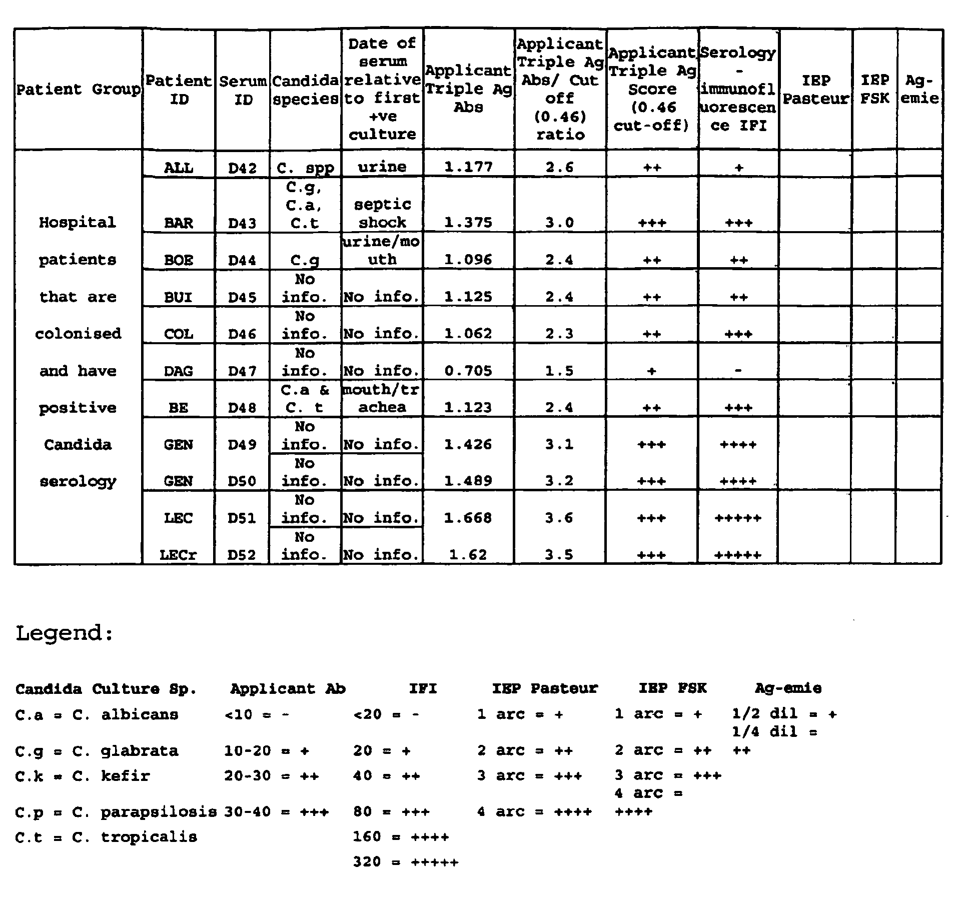

Of the 12 patients who had sera taken before or on the day of the first positive blood culture, 8 gave a positive (or low positive) result. When compared with other serology tests used by the French group 12 out of 19 patients were positive by the immunofluorescence (IFI) serology test. All but one of these was positive using the Applicant antigen test. One patient was also positive by the Applicant antigen test, but negative by IFI. All of the 5 patients that tested positive by IEP Pasteur, IEP FSK or Ag-emie serology tests were also positive by the Applicant antigen test.

It is possible that some of the patients that were negative by both the Applicant and the IFI test may have had a transient candidemia due to central line contaminations.

Six of 11 patients that were know to be colonised, but had negative serology were positive by the Applicant antigen test. Two of the positive patients were also positive by IFI. Of the 10 non-colonised hospital patients with negative serology six were positive by the Applicant antigen test, two of these positive patients were also positive by IFI. All nine patients that were colonised patients with positive serology were positive by the Applicant antigen test. These data compared to 8 out of 9 patients that were positive by IFI. The only IFI negative sample was a low positive by the Applicant antigen test.

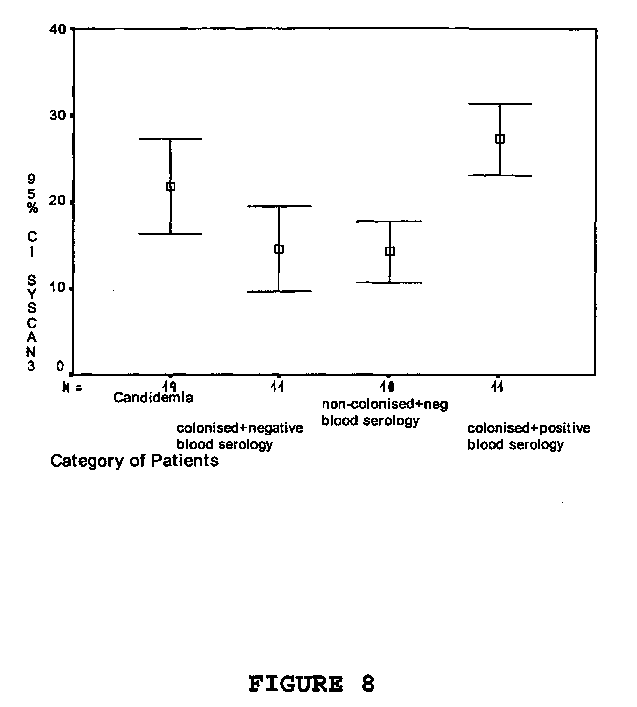

The statistical analysis of these data is presented in Figure 8 and Table 2.

**TABLE 2**

| **Category** | **Mean (Units)** | **95% Confidence Interval** |
|---|---|---|
| Candidemia Patients | 21.79^{a,b} | 16.25 - 27.33 |
| Colonised + negative Serology | 14.55^{a,c} | 9.67 - 19.42 |
| Non-colonised + negative serology | 14. 2^{b,d} | 10.68 - 17.72 |
| Colonised + positive serology | 27.27^{c,d} | 23.12 - 31.43 |

| | | |
|---|---|---|
| a. p = 0.71 b. p = 0.58 c & d. p <0.01 | | |

In the candidemia patients with positive blood culture to *Candida,* the mean of their *Candida* antibody levels detected by Applicant antigen test was 21.79 (16.25 - 27.33 95% CI). Using the Independent Samples T-test, the p value was 0.71 between the candidemia group and the colonised group with negative serology to *Candida.* The p value was 0.58 between the means of the candidemia group and the non-colonised group that was negative for *Candida* serology.

For the negative blood culture patients, the patients in the groups that were negative for *Candida* serology had generally lower *Candida* antibody levels detected by the Applicant antigen test. The mean antibody levels were 14.55 units in the colonised group (9.67 - 19.42, 95% CI) and 14.2 units in the non-colonised group (10.68-17.72, 95% CI). These levels were significantly lowered (p<0.01) from the mean antibody levels in the group of patients that had positive *Candida* colonisation culture results and positive *Candida* serology, mean 27.27 (23.12-31.43, 95% CI). These are clearly seen in the error bars in Figure 8.

Overall, there was a good correlation with the Applicant antigen test and other tests used. There was also a good correlation with the titre of antibody detected by the Applicant antigen test and the level of positiveness of the other tests ie., a patient that had a high positive result with the Applicant antigen test also had a similar result with the other tests used (eg, patients AMI, COM, DA SI, FON, PAS and RAM). Similarly patients that were negative or low positive with the Applicant antigen test were also negative or weak positive by the other tests (eg, patients BRIG, FER, HEN, LON and MAN). It was noted that some of the blood culture negative patients were positive by the Applicant antigen test, which demonstrated the great sensitivity of the Applicant antigen test.

### EXAMPLE 4 CLINICAL EVALUATION IN SPAIN

A similar clinical evaluation to that undertaken in Example 3 was conducted by Professors Guillermo Quindós, MD, PhD, María Dolores Moragues, PhD, and José Pontón, PhD, Department of Immunology, Microbiology and Parasitology at the Faculty of Medicine, University of País Vasco, Bilbao, Spain.

The retrospective study sera were obtained from 11 patients (Table 3 - Patients 1.1 to 1.32) with invasive candidiasis as defined by positive blood culture or by histology and positive tissue biopsy. The "blood culture negative" group consisted of sera from 12 patients (Table 4 - Patients 2.2 to 2.53) selected on the basis of the patients have a risk of invasive candidiasis but having negative blood cultures. Between 3 and 5 sera were tested per patient. For patients with microbiologically proven candidiasis the sera were taken before, at the time of and after the positive blood culture. For the blood culture negative group the sera were taken at various times during hospitalisation. As well as sera from hospital patients, sera from three healthy blood donors were also tested (Table 5). Also a group of fresh sera were prospectively tested from 5 patients, two with positive *Candida* blood cultures and 3 without (Table 5).

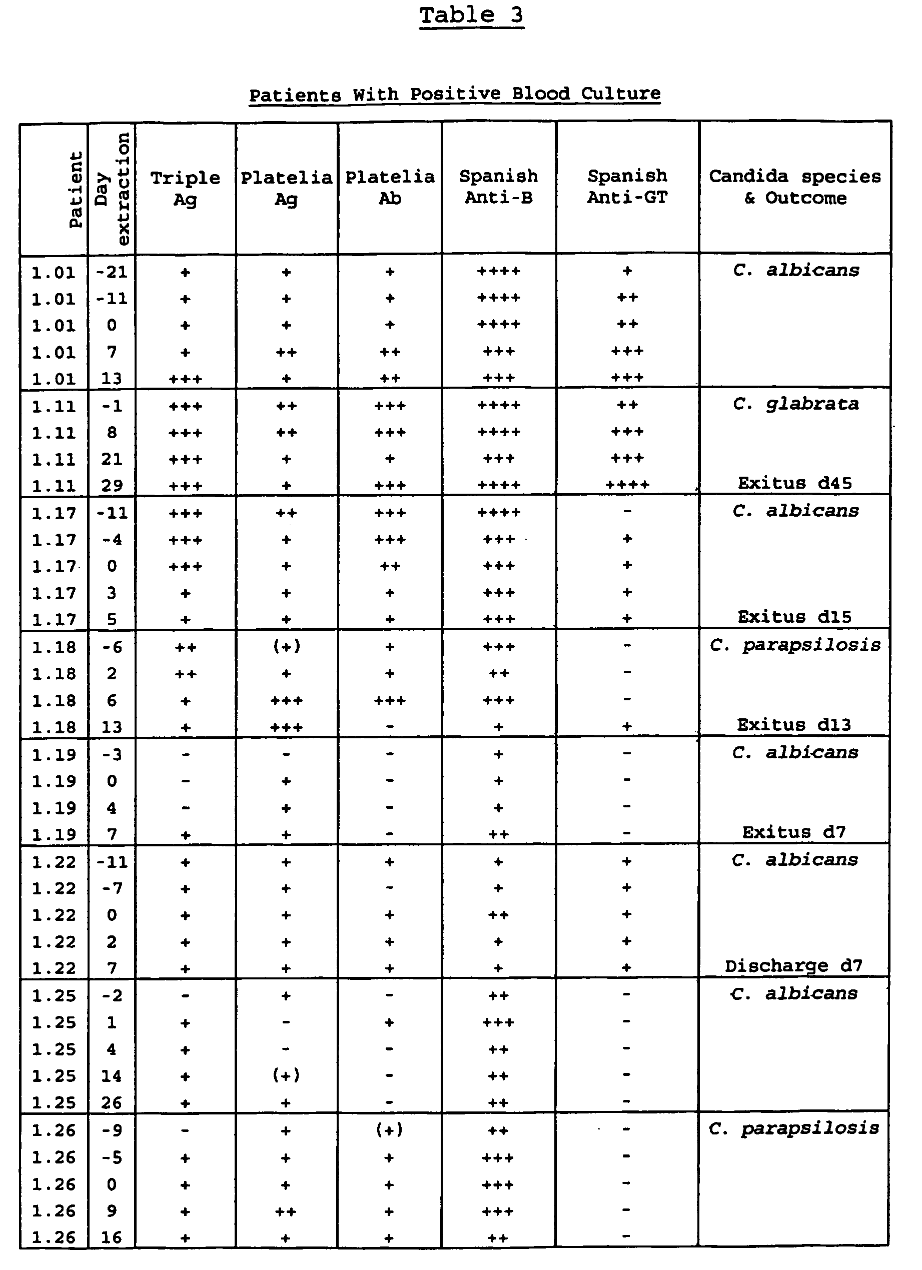

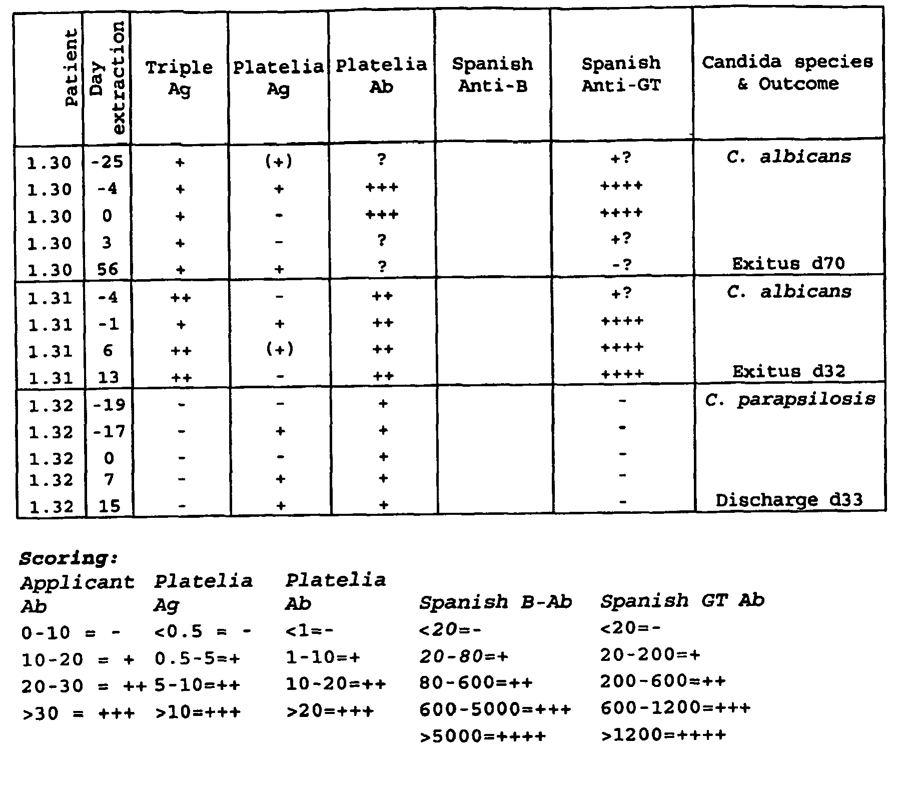

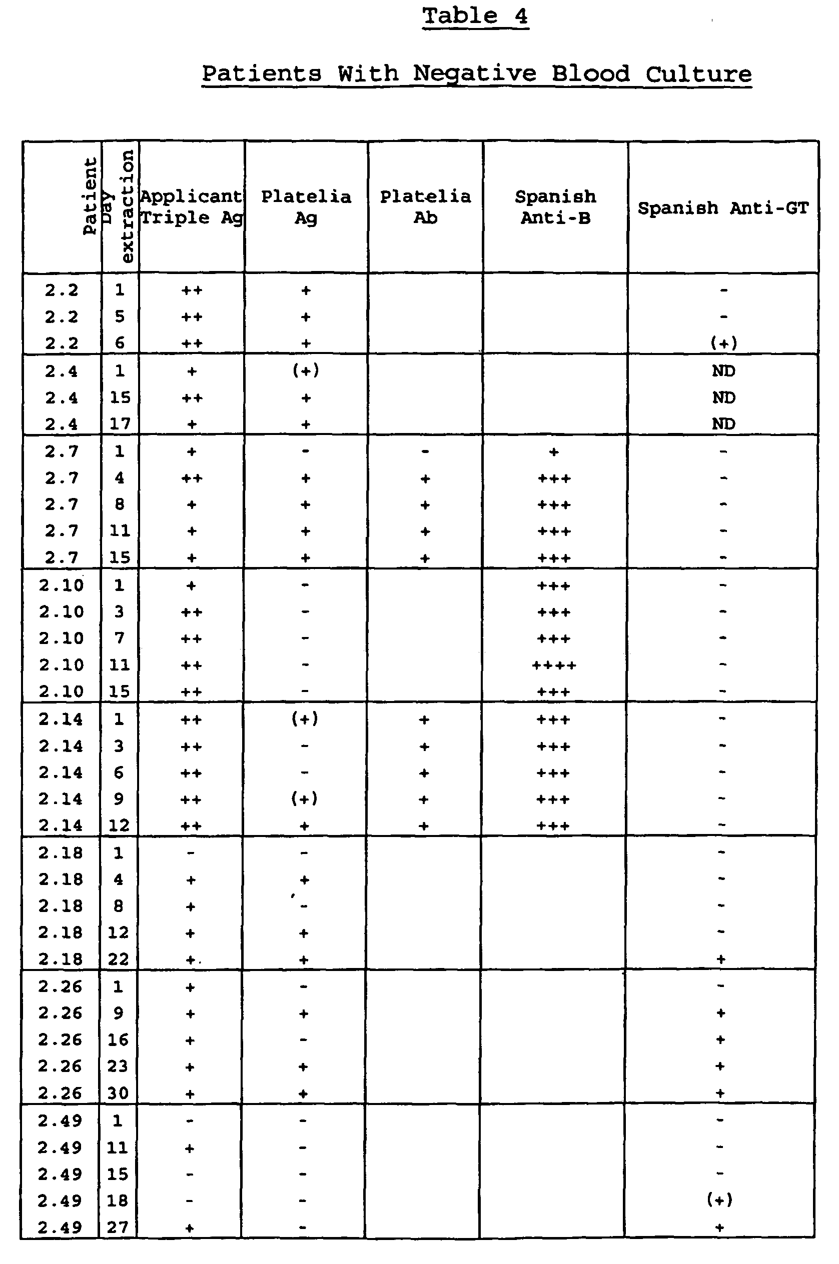

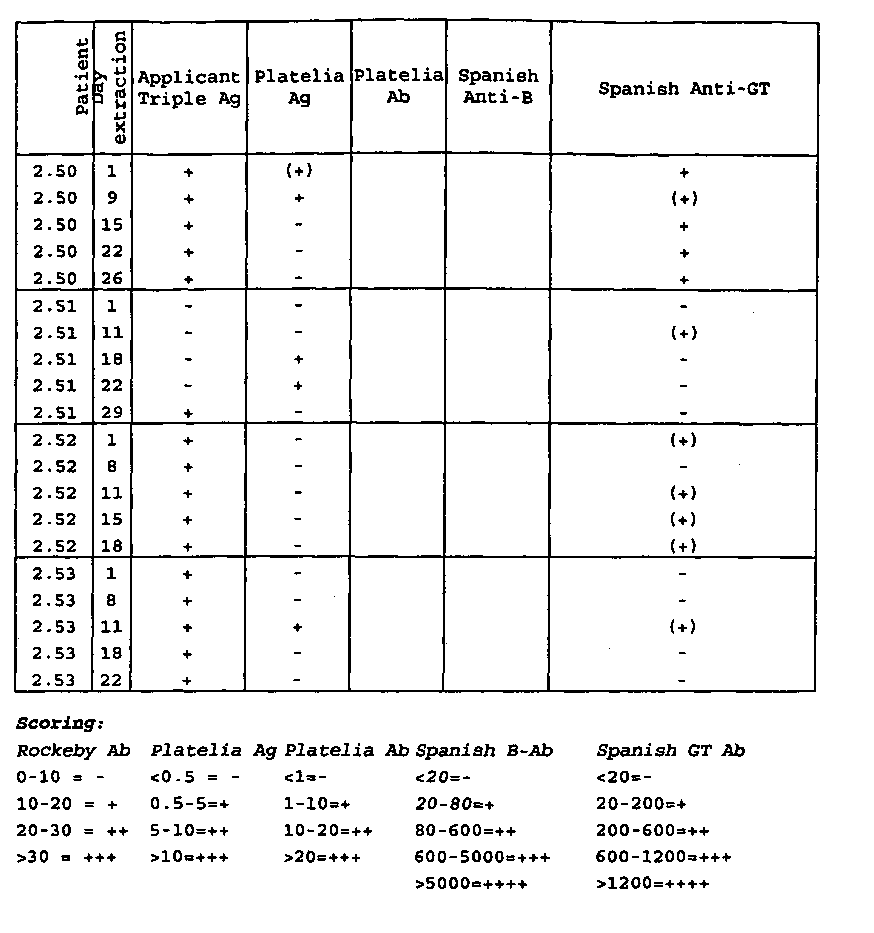

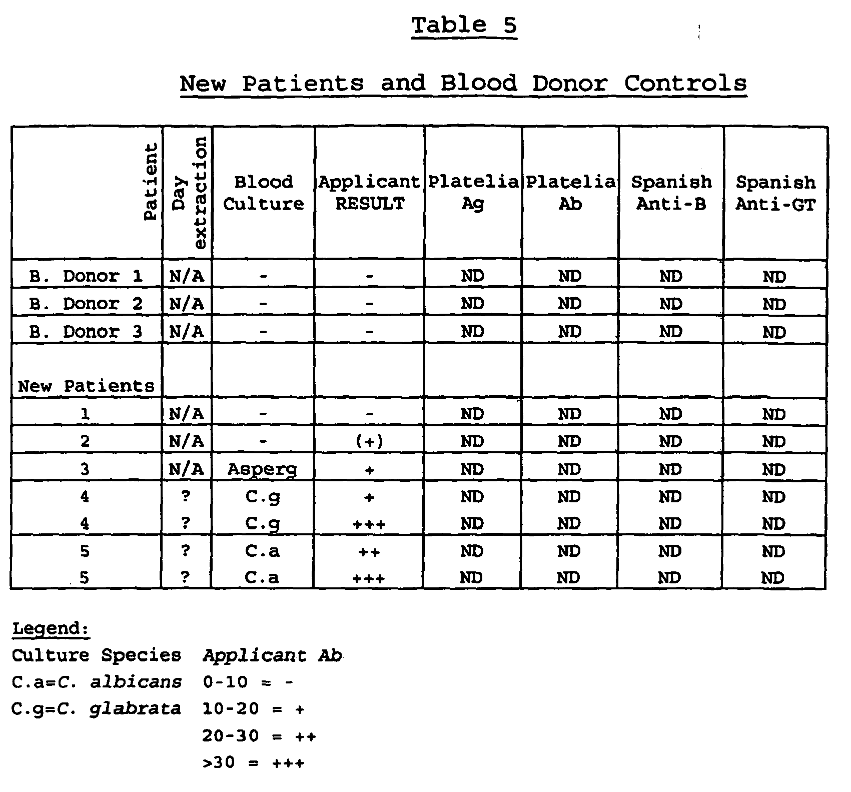

Table 6 summarises the original Spanish data split into the two groups of patients, one blood culture positive and the other blood culture negative. Of the blood culture positive group, the Applicant antigen test identified 8/11 patients as positive before they became blood culture positive. Ultimately 10/11 patients were positive with the Applicant antigen test. Only one patient (1.32) remained negative. This patient was also negative by the Spanish germ tube antibody test and was only transiently positive by the Platelia (BioRad) mannan antigen test. It may be possible that this patient had a transient candidemia.

**Table 6**

| Summary of Spanish Data | | | | | |
|---|---|---|---|---|---|
| | Applicant Triple Ag | Platelia Ag | Platelia Ab | Spanish Anti-B | Spanish Anti-GT |
| Culture Positive Patients | | | | | |
| Negative result | 1 | 0 | 1 | 0 | 4 |
| Positive before culture | 8 | 11 | 9 | 8 | 6 |
| Positive after culture | 2 | | 1 | 0 | 1 |
| Total Patients tested | 11 | 11 | 11 | 8 | 11 |

| | | | | | |
|---|---|---|---|---|---|
| Culture Negative Patients | | | | | |
| Negative result | 0 | 3 | 0 | 0 | 6 |
| Positive result | 12 | 9 | 2 | 3 | 5 |
| Total Patients tested | 12 | 12 | 2 | 3 | 11 |

| | | | | | |
|---|---|---|---|---|---|
| Patient ID | | | | | |
| 1.01 | pos | pos | pos | pos | pos |
| 1.11 | pos | pos | pos | pos | pos |
| 1.17 | pos | pos | pos | pos | pos |
| 1.18 | pos | pos | pos | pos | (pos) |
| 1.19 | (pos) | pos | neg | pos | neg |
| 1.22 | pos | pos | pos | pos | pos |
| 1.25 | pos | pos | (pos) | pos | neg |
| 1.26 | pos | pos | pos | pos | neg |
| 1.30 | pos | pos | pos | ND | pos |
| 1.31 | pos | pos | pos | ND | pos |
| 1.32 | neg | pos | pos | ND | neg |

| | | | | | |
|---|---|---|---|---|---|
| Patient ID | | | | | |
| 2.02 | pos | pos | | | neg |
| 2.04 | pos | pos | | | |
| 2.07 | pos | pos | pos | pos | neg |
| 2.10 | pos | neg | | pos | neg |
| 2.14 | pos | (pos) | pos | pos | neg |
| 2.18 | pos | pos | | | (pos) |
| 2.26 | pos | pos | | | pos |
| 2.49 | pos | neg | | | (pos) |
| 2.50 | pos | (pos) | | | pos |
| 2.51 | (pos) | pos | | | neg |
| 2.52 | pos | neg | | | neg |
| 2.53 | pos | (pos) | | | neg |

The Applicant antigen test identified all 12 of the blood culture negative patient group as being positive for *Candida* antibody. In comparison, the Platelia Mannan antigen test identified 9/12 patients as being positive compared to 5/11 by the Spanish Germ tube antibody test. The main problem with the blood culture negative patient group was that there was no other confirmation of diagnosis. overall, there was good correlation with the results of the Applicant antigen test and that of the other serology tests used by the Spanish group. Where a patient was strongly positive by the Applicant antigen test (ie.: patient's 1.11, 1.17, 1.18 and 1.31), they were also strongly positive by the other tests. Also, where sera were negative or low positive by the Applicant antigen test, they are also usually negative or weakly positive by the other tests. For example, patients 1.19, 1.22, 1.25, 1.26, 1.32, 2.18, 2.26, 2.49, 2.50, 2.51, 2.52 and 2.53.

Where fresh sera was analysed (Table 5), there was a perfect correlation with the Applicant antigen test and whether the sera was blood culture positive or negative.

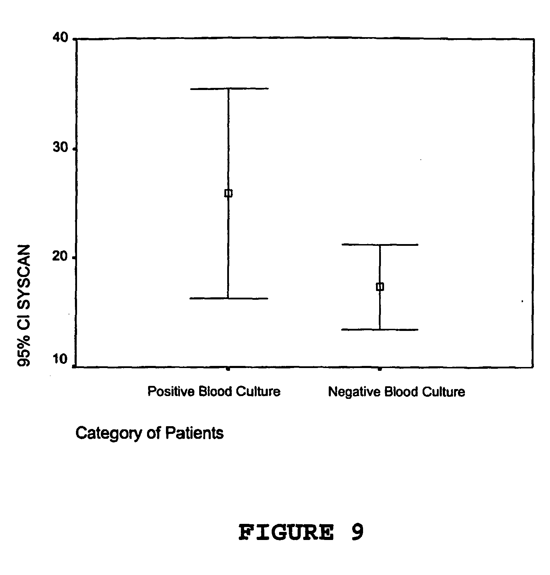

The statistical analysis of these data is presented in Figure 9 and Table 7. From the error plot diagram in Figure 9, it is evident that the group of patients with positive blood culture have a higher *Candida* antibody levels detected by Syscan3 (mean 25.86, 95% CI: 16.28 - 35.44) as compared to the patients with negative blood culture as a group (mean 17.30, 95% CI: 13.42 - 21.19). Comparing the means using the Independent Samples T-Test, the difference between the two groups is statistically significant at p = 0.087.

**Table 7**

| Mean and 95% Confidence Interval of Mean of Applicant Antigen Test Scores | | |
|---|---|---|
| Category | Mean (Units) | 95% Confidence Interval |
| Positive Blood Culture | 25.86* | 16.28 - 35.44 |
| Negative Blood Culture | 17.30* | 13.42 - 21.19 |

| | | |
|---|---|---|
| * p = 0.087 | | |

### EXAMPLE 5 CLINICAL EVALUATION IN AUSTRALIA

Sera collected from patients with invasive candidiasis was obtained from an Australian hospital (1997 to 1998), the patients had haematological malignancies (n=24). Control sera were collected from males 18 to 25 years of age (n=20) with no history of *Candida* infection. The patient sera were tested with the Applicant antigen test as described in Example 2. Each sera was tested in triplicate and the average reading used. The average absorbance reading for each serum was divided by that of the "cut-off" calibrator serum supplied with the Applicant antigen test. This value was then multiplied by 10 to give a value in arbitrary units.

The results of the Applicant antigen test using a value of 20 units (two times the cut-off calibrator serum value) or above as defining a positive sample is presented in Table 8.

**Table 8**

| Results of the Applicant antigen test using 20 units as a cut-off | | | |
|---|---|---|---|
| | Invasive Candidiasis | Healthy Controls | *Total* |
| *Test Positive* | 20 | 0 | 20 |
| *Test Negative* | 4 | 20 | 24 |
| Total | 24 | 20 | 44 |

With the Applicant antigen test using 20 units as cut-off, the specificity of the test was 100% and sensitivity was 83.3%. -Positive predictive value was 83.3% and negative predictive value was 100%. When the value of the test considered positive was set at 10 units or 1 times the value of the cut-off sera absorbance, the specificity of the test decreased, but the sensitivity increased (Table 9). The specificity was 90%, sensitivity 87.5%. Positive predictive factor increased to 91.3%, while negative predictive factor decreased to 85.7%.

**Table 9**

| Results of the Applicant antigen test using 10 units as a cut-off | | | |
|---|---|---|---|
| | Invasive Candidiasis | Healthy Controls | *Total* |
| *Test Positive* | 21 | 2 | 23 |
| *Test Negative* | 3 | 18 | 21 |
| Total | 24 | 20 | 44 |

The results of the Applicant antigen test using sera from patients with invasive candidiasis are presented in Table 10. Only one of the negative results came from a patient who was blood culture positive. Two of the four negative samples were from patients with central line contaminations. Three of the four negative test result came from patients with *Candida parapsilosis* infections, the other being *C. albicans.*

**Table 10**

| Sera from Patients with Invasive Candidiasis Test with Applicant Antigen Test | | | | | |
|---|---|---|---|---|---|
| Patient | Abs (420 nm) | Units | Result | Site of isolation | Candida spp. |
| A | 1.69 | 61 | Positive | blood culture | parapsilosis |
| B | 0.15 | 5 | Negative | blood culture | parapsilosis |
| C | 1.16 | 42 | Positive | peritoneal cavity | guillermondii |
| D | 1.85 | 67 | Positive | blood culture | albicans |
| E | 1.16 | 42 | Positive | blood culture | albicans |
| F | 1.49 | 54 | Positive | peritoneal cavity | glabrata |
| G | 0.56 | 20 | Positive | peritoneal cavity | parapsilosis |
| I | 1.24 | 45 | Positive | cathater | albicans |
| K | 0.98 | 35 | Positive | oesophagus | albicans |
| L | 3.46 | 124 | Positive | peritoneal cavity | albicans |
| M | 0.19 | 7 | Negative | central line | albicans |
| N | 1.44 | 52 | Positive | wound | albicans |
| P | 0.55 | 20 | Positive | sputum | glabrata |
| Q | 1.12 | 40 | Positive | sputum | tropicalis |
| R | 1.02 | 37 | Positive | central line | albicans |
| S | 0.88 | 32 | Positive | blood culture | albicans |
| T | 1.6 | 58 | Positive | blood culture | glabrata |
| U | 0.22 | 8 | Negative | central line | parapsilosis |
| V | 0.55 | 20 | Positive | urine | parapsilosis |
| W | 0.31 | 11 | Negative | peritoneal cavity | parapsilosis |
| X | 0.59 | 21 | Positive | central line | albicans |
| Y | 0.85 | 31 | Positive | blood culture | albicans |
| Z | 1.06 | 38 | Positive | bronch | albicans |
| ZA | 1.05 | 38 | Positive | urine | tropicalis |

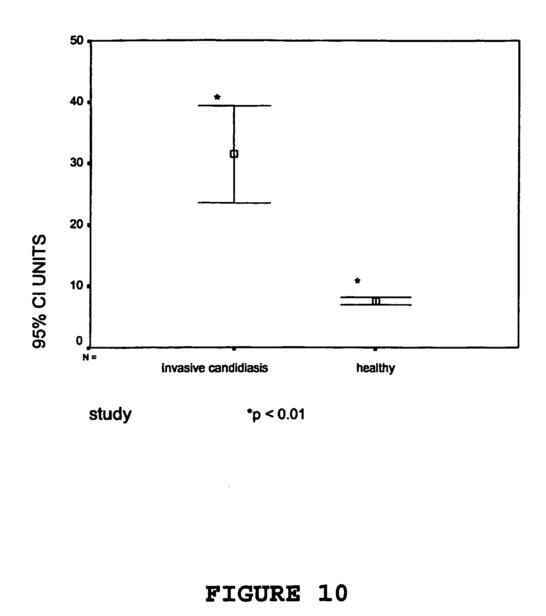

The Applicant antigen test data for the invasive candidiasis group and healthy controls are presented in the error bar diagram as seen in Figure 10 and Table 11. In Figure 10, the group with invasive candidiasis has a higher mean (31.45 units) as compared to the healthy blood donor group (7.52 units). This difference was statistically significant (p < 0.01). The 95% confidence interval range of the means was higher as well in the invasive candidiasis group (23.57 - 39.33 units), as compared to the healthy donor group (6.92 - 8.12 units).

**Table 11**

| Mean and 95% Confidence Interval of the Mean of Patients with Invasive Candidiasis and healthy blood donors | | |
|---|---|---|
| Group | Mean (Units) | 95% CI of mean(Units) |
| Invasive Candidiasis | 31.45 | 23.57 - 39.33 |
| Healthy blood donors | 7.52 | 6.92 - 8.12 |

In this study the Applicant antigen test was used to test sera from patients with invasive candidiasis, superficial candidiasis (oral or vaginal thrush) and healthy male controls. As a commensal organism, healthy individuals can have a measurable antibody titre to *Candida* antigens. In order to differentiate between normal and infection associated antibody levels a cut-off calibrator serum was supplied. The absorbance of the serum being tested was divided by the cut-off calibrator serum absorbance and multiplied by 10 to give an arbitrary unit value. Using a value of 20 units or above as an indicator of a positive test gave the greatest discrimination between the patient group with invasive candidiasis and the healthy controls (positive predictive value of 83%, negative predictive value 100%). If the value at which a sample was considered positive was lowered to 10 units (ie. the cut-off calibrator value), the positive predictive value increased slightly to 87.5% but the negative predictive value decreased to 90%.

Only one patient with a positive blood culture returned a negative test result with the Applicant antigen test. Two out of the four negative sera were from patients with a central line contamination. This could therefore reflect a transient infection in these patients, which may not provoke an antibody response. It is of interest that 3 of the 4 negative tests were due to C. *parapsilosis* infections. This organism is frequently associated with biofilms, which may shield it from the host immune response.

In conclusion, the Applicant antigen test is a rapid, reliable and easy test to perform. It showed good sensitivity and specificity in the diagnosis of invasive and severe superficial *Candida* infections.

## Claims

1. A method of diagnosing *Candida albicans* infection, comprising the steps of:
a) preparing an antigen composition by a method comprising filtration and organic extraction of yeast cells of *Candida albicans* to produce a soluble cytoplasmic protein fraction, the antigen composition being mannose depleted and comprising antigens of molecular weights 55 kDa, 30 kDa, 20 kDa and four of 35 to 45 kDa (when determined by SDS-PAGE) for detecting antibodies to *Candida albicans;*
b) contacting said antigen composition with a biological sample obtained from a subject at risk of, or suspected to be suffering from, *Candida albicans* infection; and
c) using a detection system to determine if antibodies from the biological sample are bound to said antigen composition by means of an antigen/antibody reaction.

2. A method according to claim 1, wherein the antigen composition further comprises one or more antigens selected from the group consisting of cell wall and enolase antigen.

3. A method according to claim 1 or claim 2, wherein step c) is a detection system selected from the group consisting of enzyme-linked immunoassay (ELISA or EIA), biligand binding (sandwich technique), fluorometric assay, chemiluminescent assay, radialimmunodiffusion and radioimmunoassay (RIA).

4. A method according to claim 1 or claim 2, wherein step c) is by ELISA or chemiluminescent assay.

5. A method according to any one of claims 1 to 4, further comprising the step of binding the antigen composition to a solid phase either by absorptive binding, covalent binding, or via a ligand already bound to the solid phase.

6. A method according to any one of claims 1 to 5, further comprising the step of using secondary labelled antibodies to detect the antibodies to *Candida albicans* present in the biological samples.

7. A method according to claim 6, further comprising the step of labelling the secondary antibodies with a label selected from the group consisting of fluorescent dye, radioisotope and enzyme, or combinations thereof.

8. A method according to claim 7, wherein the secondary antibody is labelled via bound ligands.

9. A method according to claim 1, wherein detection in the detection system is selected from the group consisting of colour development, chemiluminescence, fluorescence, radioactivity, or combinations thereof.

10. A method according to any one of claims 1 to 9, further comprising the step of performing the detection of antibodies by a method selected from the group consisting of qualitative detection and quantitative detection, or combination thereof.

11. A method according to claim 7, further comprising the step of directly labelling the secondary antibody.

12. A method according to claim 7, further comprising the step of indirectly labelling the secondary antibody.

13. A method according to any one of claims 1 to 12, wherein the antigen composition is either immobilised on an inert surface, embedded in a gel, or conjugated to a molecule which imparts colour, fluorescence or radioactivity to the antigen.

14. A method according to any one of claims 1 to 13, wherein the biological sample is selected from the group consisting of bone marrow, plasma, spinal fluid, lymph fluid, the external sections of the skin from respiratory, intestinal and genitourinary tracts, tears, saliva, milk, blood; both whole blood and serum, blood cells, tumours and organs.

15. A method according to claim 14, wherein the biological sample is serum.

16. A antigen composition prepared by a method comprising filtration and organic extraction of yeast cells of *Candida albicans* to produce a soluble cytoplasmic protein fraction, the antigen composition being mannose depleted and comprising antigens of molecular weights 55 kDa, 30 kDa, 20 kDa and four of 35 to 45 kDa (when determined by SDS-PAGE) for detecting antibodies to *Candida albicans.*

17. A method of any one of claims 1 to 15 or composition of claim 16, wherein the organic extraction is chloroform extraction.

## Patentansprüche

1. Verfahren zum Diagnostizieren einer Infektion mit *Candida albicans* mit den Stufen, bei denen man:
a) eine Antigenzusammensetzung nach einem Verfahren erzeugt, welches das Filtrieren und die organische Extraktion von Hefezellen von *Candida albicans* umfasst, um eine Fraktion löslichen zytoplasmatischen Proteins zu erzeugen, wobei die Antigenzusammensetzung Mannose-abgereichert ist und Antigene mit den Molekulargewichten 55 kDa, 30 kDa, 20 kDa und vier mit 35 bis 45 kDa (wenn mit SDS-PAGE bestimmt) zum Detektieren von Antikörpern gegen *Candida albicans* umfasst;
b) die Antigenzusammensetzung mit einer biologischen Probe, die aus einem Patienten mit dem Risiko einer oder dem Verdacht des Leidens unter einer Infektion mit *Candida albicans* erhalten wurde, in Kontakt bringt und
c) ein Detektionssystem verwendet, um zu bestimmen, ob Antikörper aus der biologischen Probe mittels einer Antigen/Antikörper-Reaktion an die Antigenzusammensetzung gebunden werden.

2. Verfahren nach Anspruch 1, wobei die Antigenzusammensetzung weiterhin eines oder mehrere Antigene umfasst, die ausgewählt sind aus der Gruppe, bestehend aus Zellwand- und Enolase-Antigen.

3. Verfahren nach Anspruch 1 oder Anspruch 2, wobei in Stufe c) das Detektionssystem ausgewählt ist aus der Gruppe, bestehend aus einem Enzym-gekoppelten Immuntest (ELISA oder EIA), Biliganden-Bindung (Sandwich-Verfahren), Fluorimetrietest, Chemolumineszenztest, Radial-Immunodiffusions- und Radioimmuntest (RIA).

4. Verfahren nach Anspruch 1 oder Anspruch 2, wobei die Stufe c) mit ELISA oder einem Chemolumineszenztest durchgeführt wird.

5. Verfahren nach einem der Ansprüche 1 bis 4, welches weiterhin die Stufe umfasst, bei der man die Antigenzusammensetzung entweder durch Absorptionsbindung, kovalente Bindung oder über einen bereits an die Festphase gebundenen Liganden an eine Festphase bindet.

6. Verfahren nach einem der Ansprüche 1 bis 5, welches weiterhin die Stufe umfasst, bei der man sekundär markierte Antikörper verwendet, um die Antikörper gegen *Candida albicans,* die in der biologischen Probe vorliegen, zu erfassen.

7. Verfahren nach Anspruch 6, welches weiterhin die Stufe umfasst, bei der man die sekundären Antikörper mit einer Markierung markiert, die ausgewählt ist aus der Gruppe, bestehend aus einem Fluoreszenzfarbstoff, einem Radioisotop und einem Enzym oder Kombinationen davon.

8. Verfahren nach Anspruch 7, wobei der sekundäre Antikörper über gebundene Liganden markiert ist.

9. Verfahren nach Anspruch 1, wobei die Detektion in dem Detektionssystem ausgewählt ist aus der Gruppe, bestehend aus einer Farbentwicklung, Chemoluminenszenz, Fluoreszenz, Radioaktivität oder Kombinationen davon.

10. Verfahren nach einem der Ansprüche 1 bis 9, welches weiterhin die Stufe umfasst, bei der man die Detektion von Antikörpern nach einem Verfahren durchführt, welches ausgewählt ist aus der Gruppe, bestehend aus qualitativer Detektion und quantitativer Detektion oder einer Kombination davon.

11. Verfahren nach Anspruch 7, welches weiterhin die Stufe umfasst, bei der man den sekundären Antikörper unmittelbar markiert.

12. Verfahren nach Anspruch 7, welches weiterhin die Stufe umfasst, bei der man den sekundären Antikörper indirekt markiert.

13. Verfahren nach einem der Ansprüche 1 bis 12, wobei die Antigenzusammensetzung entweder auf einer inerten Oberfläche immobilisiert ist, in einem Gel eingebettet ist oder mit einem Molekül, welches dem Antigen Farbe, Fluoreszenz oder Radioaktivität verleiht, konjugiert ist.

14. Verfahren nach einem der Ansprüche 1 bis 13, wobei die biologische Probe ausgewählt ist aus der Gruppe, bestehend aus Knochenmark, Plasma, Zerebrospinalflüssigkeit, Lymphflüssigkeit, den äußeren Abschnitten der Haut der Atemwege, des Intestinal- und des Urogenitaltrakts, Tränenflüssigkeit, Speichel, Milch, Blut, sowohl Vollblut als auch Serum, Blutzellen, Tumore und Organe.

15. Verfahren nach Anspruch 14, wobei die biologische Probe Serum ist.

16. Antigenzusammensetzung, hergestellt nach einem Verfahren umfassend das Filtrieren und die organische Extraktion von Hefezellen von *Candida albicans,* um eine Fraktion an löslichem zytoplasmatischen Protein zu erzeugen, wobei die Antigenzusammensetzung Mannose-abgereichert ist und Antigene mit den Molekulargewichten 55 kDa, 30 kDa, 20 kDa und vier mit 35 bis 45 kDa (wenn anhand von SDS-PAGE bestimmt) umfasst, für die Detektion von Antikörpern gegen *Candida albicans.*

17. Verfahren nach einem der Ansprüche 1 bis 15 oder Zusammensetzung nach Anspruch 16, wobei die organische Extraktion eine Chloroformextraktion ist.

## Revendications

1. Méthode pour le diagnostic d'une infection par *Candida albicans,* comprenant les étapes consistant à :
a) préparer une composition d'antigène par un procédé comprenant la filtration et l'extraction avec un agent organique de cellules de la levure *Candida albicans* pour produire une fraction de protéines cytoplasmiques solubles, la composition d'antigène étant appauvrie en mannose et comprenant des antigènes ayant des poids moléculaires de 55 kDa, 30 kDa, 20 kDa et quatre de 35 à 45 kDa (lors de la détermination par SDS-PAGE) pour la détection d'anticorps contre *Candida albicans ;*
b) mettre en contact ladite composition d'antigènes avec un échantillon biologique obtenu chez un sujet présentant un risque, ou supposé souffrir, d'une infection par *Candida albicans ;* et
c) utiliser un système de détection pour déterminer si les anticorps de l'échantillon biologique sont liés à ladite composition d'antigènes au moyen d'une réaction antigène/anticorps.

2. Méthode suivant la revendication 1, dans laquelle la composition d'antigènes comprend en outre un ou plusieurs antigènes choisis dans le groupe consistant en des antigènes de paroi cellulaire et d'énolase.

3. Méthode suivant la revendication 1 ou la revendication 2, dans laquelle l'étape c) est un système de détection choisi dans le groupe consistant en une analyse immunologique par liaison à une enzyme (ELISA ou EIA), une liaison de ligand double (technique en sandwich), une analyse fluorimétrique, une analyse chimioluminescente, l'immunodiffusion radiale et une analyse radio-immunologique (RIA).

4. Méthode suivant la revendication 1 ou la revendication 2, dans laquelle l'étape c) est mise en oeuvre par une analyse ELISA ou analyse chimioluminescente.

5. Méthode suivant l'une quelconque des revendications 1 à 4, comprenant en outre l'étape de liaison de la composition d'antigène à une phase solide par liaison par absorption, liaison covalente ou bien par l'intermédiaire d'un ligand déjà lié à la phase solide.

6. Méthode suivant l'une quelconque des revendications 1 à 5, comprenant en outre l'étape d'utilisation d'anticorps secondaires marqués pour détecter les anticorps contre *Candida albicans* présents dans les échantillons biologiques.

7. Méthode suivant la revendication 6, comprenant en outre l'étape de marquage des anticorps secondaires avec un marqueur choisi dans le groupe consistant en un colorant fluorescent, un radio-isotope, une enzyme ou leurs associations.

8. Méthode suivant la revendication 7, dans laquelle l'anticorps secondaire est marqué par l'intermédiaire de ligands liés.

9. Méthode suivant la revendication 1, dans laquelle la détection dans le système de détection est choisie dans le groupe consistant en un développement de couleurs, la chimioluminescence, la fluorescence, la radioactivité ou leurs associations.

10. Méthode suivant l'une quelconque des revendications 1 à 9, comprenant en outre l'étape de mise en oeuvre de la détection des anticorps par une méthode choisie dans le groupe consistant en la détection qualitative et la détection quantitative, ou leur association.

11. Méthode suivant la revendication 7, comprenant en outre l'étape de marquage direct de l'anticorps secondaire.

12. Méthode suivant la revendication 7, comprenant en outre l'étape de marquage indirect de l'anticorps secondaire.

13. Méthode suivant l'une quelconque des revendications 1 à 12, dans laquelle la composition d'antigènes est immobilisée sur une surface inerte, incluse dans un gel, ou bien conjuguée à une molécule qui confère une couleur, une fluorescence ou une radioactivité à l'antigène.

14. Méthode suivant l'une quelconque des revendications 1 à 13, dans laquelle l'échantillon biologique est choisi dans le groupe consistant en la moelle osseuse, le plasma, le liquide rachidien, le liquide lymphatique, les sections externes de la peau, du tractus respiratoire, du tractus intestinal et du tractus génito-urinaire, les larmes, la salive, le lait, le sang, le sang entier et le sérum, des cellules sanguines, des tumeurs et des organes.

15. Méthode suivant la revendication 14, dans laquelle l'échantillon biologique est un échantillon de sérum.

16. Composition d'antigènes préparée par un procédé comprenant la filtration et l'extraction avec un agent organique de cellules de la levure *Candida albicans* pour produire une fraction de protéines cytoplasmiques solubles, la composition d'antigènes étant appauvrie en mannose et comprenant des antigènes ayant des poids moléculaires de 55 kDa, 30 kDa, 20 kDa et quatre de 35 à 45 kDa (lors de la détermination par SDS-PAGE) pour la détection d'anticorps contre *Candida albicans.*

17. Méthode suivant l'une quelconque des revendications 1 à 15 ou composition suivant la revendication 16, dans laquelle l'extraction avec un agent organique est une extraction au chloroforme.
